(19) Europäisches Patentamt
European Patent Office
Office européen des brevets

(11) EP 1 454 627 A1

(12) **EUROPEAN PATENT APPLICATION**

(43) Date of publication:
**08.09.2004 Bulletin 2004/37**

(21) Application number: **03004910.0**

(22) Date of filing: **06.03.2003**

(51) Int Cl.[7]: **A61K 38/05**, A61K 38/06, C07D 339/04, C07K 5/062, C07C 237/22, C07D 233/54, C07K 5/023, C07K 5/072, A61P 25/28, A61P 21/00

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK RO**

(71) Applicant: **MyoContract Ltd.**
**4410 Liestal (CH)**

(72) Inventors:
- **Henneböhle, Marco**
  **79618 Rheinfelden (DE)**
- **Herzner, Holger**
  **4434 Hölstein (CH)**
- **Lescop, Cyrille**
  **68680 Kembs Loechle (FR)**
- **Siendt, Herbé**
  **68330 Huningue (FR)**
- **Weyermann, Philipp**
  **4450 Sissach (CH)**
- **von Sprecher, Andreas**
  **4104 Oberwil (CH)**

(74) Representative: **Grünecker, Kinkeldey,**
**Stockmair & Schwanhäusser Anwaltssozietät**
**Maximilianstrasse 58**
**80538 München (DE)**

# (54) Alpha-Keto carbonyl calpain inhibitors

(57) The present invention relates to novel α-keto carbonyl calpain inhibitors for the treatment of neurodegenerative diseases and neuromuscular diseases including Duchenne Muscular Dystrophy, Becker Muscular Dystrophy and other muscular dystrophies. Disuse atrophy and general muscle wasting can also be treated. Generally all conditions where elevated levels of calpains are involved can be treated. The compounds of the invention may also inhibit other thiol proteases such as cathepsin B, cathepsin H, cathepsin L, papain or the like. Multicatalytic Protease (MCP) also known as proteasome may also be inhibited. The compounds of the present invention are also inhibitors of cell damage by oxidative stress through free radicals and can be used to treat mitochondrial disorders and neurodegenerative diseases, where elevated levels of oxidative stress are involved.

EP 1 454 627 A1

## Description

### Field of the Invention

The present invention relates to novel α-keto carbonyl calpain inhibitors for the treatment of neurodegenerative diseases and neuromuscular diseases including Duchenne Muscular Dystrophy, Becker Muscular Dystrophy and other muscular dystrophies. Disuse atrophy and general muscle wasting can also be treated. Generally all conditions where elevated levels of calpains are involved can be treated.

The novel calpain inhibitors may also inhibit other thiol proteases, such as cathepsin B, cathepsin H, cathepsin L and papain. Multicatalytic Protease (MCP) also known as proteasome may also be inhibited by the compounds of the invention.

The compounds of the present invention are also inhibitors of cell damage by oxidative stress through free radicals and can be used to treat mitochondrial disorders and neurodegenerative diseases, where elevated levels of oxidative stress are involved.

Also provided are pharmaceutical compositions containing the same.

### Background of the Invention

Neural tissues, including brain, are known to possess a large variety of proteases, including at least two calcium-stimulated proteases, termed calpain I and calpain II. Calpains are calcium-dependent cysteine proteases present in a variety of tissues and cells and use a cysteine residue in their catalytic mechanism. Calpains are activated by an elevated concentration of calcium, with a distinction being made between calpain I or μ-calpain, which is activated by micromolar concentrations of calcium ions, and calpain II or m-calpain, which is activated by millimolar concentrations of calcium ions (P. Johnson, Int. J. Biochem. 1990, 22(8), 811-22). Excessive activation of calpain provides a molecular link between ischaemia or injury induced by increases in intra-neuronal calcium and pathological neuronal degeneration. If the elevated calcium levels are left uncontrolled, serious structural damage to neurons may result. Recent research has suggested that calpain activation may represent a final common pathway in many types of neurodegenerative diseases. Inhibition of calpain would, therefore, be an attractive therapeutic approach in the treatment of these diseases. Calpains play an important role in various physiological processes including the cleavage of regulatory proteins such as protein kinase C, cytoskeletal proteins such as MAP 2 and spectrin, and muscle proteins, protein degradation in rheumatoid arthritis, proteins associated with the activation of platelets, neuropeptide metabolism, proteins in mitosis and others which are listed in M. J. Barrett et al., Life Sci. 1991, 48, 1659-69 and K. K. Wang et al., Trends in Pharmacol. Sci. 1994, 15, 412-9. Elevated levels of calpain have been measured in various pathophysiological processes, for example: ischemias of the heart (eg. cardiac infarction), of the kidney or of the central nervous system (eg. stroke), inflammations, muscular dystrophies, cataracts of the eyes, injuries to the central nervous system (eg. trauma), Alzheimer's disease, etc. (see K. K. Wang, above). These diseases have a presumed association with elevated and persistent intracellular calcium levels, which cause calcium-dependent processes to be overactivated and no longer subject to physiological control. In a corresponding manner, overactivation of calpains can also trigger pathophysiological processes. Exemplary of these diseases would be myocardial ischaemia, cerebral ischaemia, muscular dystrophy, stroke, Alzheimer's disease or traumatic brain injury. Other possible uses of calpain inhibitors are listed in K. K. Wang, Trends in Pharmacol. Sci. 1994, 15, 412-8. It is considered that thiol proteases, such as calpain or cathepsins, take part in the initial process in the collapse of skeletal muscle namely the disappearance of Z line through the decomposition of muscular fiber protein as seen in muscular diseases, such as muscular dystrophy or amyotrophy (Taisha, Metabolism, 25, 183 (1988)). Furthermore, E-64-d, a thiol protease inhibitor, has been reported to have life-prolonging effect in experimental muscular dystrophy in hamster (Journal of Pharmacobiodynamics, 10, 678 (1987)). Accordingly, such thiol protease inhibitors are expected to be useful as therapeutic agents, for example, for the treatment of muscular dystrophy or amyotrophy.

On a cellular level elevated oxidative stress leads to cell damage and mitochondrial disorders such as Kearns-Sayre syndrome, mitochondrial encephalomyopathy-lactic-acidosis-stroke like episodes (MELAS), myoclonic epilepsy and ragged-red-fibers (MERRF), Leber hereditary optic neuropathy (LHON), Leigh's syndrome, neuropathy-ataxia-retinitis pigmentosa (NARP) and progressive extemal opthalmoplegia (PEO) summarized in Schapira and Griggs (eds) 1999. *Muscle Diseases,* Butterworth-Heinemann.

Cell damage induced by free radicals is also involved in certain neurodegenerative diseases. Examples for such diseases include degenerative ataxias such as Friedreich' Ataxia, Parkinson's disease, Huntington's disease, amyotrophic lateral sclerosis (ALS), and Alzheimer's disease (Beal MF, Howell N., Bodis-Wollner I (eds), 1997, *Mitochondria and free radicals in neurodegenerative diseases* , Wiley-Liss)

Calpain inhibitors have been described in the literature. However, these are predominantly either irreversible inhibitors or peptide inhibitors. As a rule, irreversible inhibitors are alkylating substances and suffer from the disadvantage that they react nonselectively in the organism or are unstable. Thus, these inhibitors often have undesirable side effects, such as toxicity, and are therefore of limited use or are unusable. Examples of the irreversible inhibitors are E-64 epoxides (E. B. McGowan et al., Biochem.Biophys.Res.Commun. 1989, 158, 432-5), alpha-haloketones (H. Angliker et al., J.Med.Chem. 1992, 35, 216-20) and disulfides (R. Matsueda et al., Chem.Lett. 1990, 191-194).

Many known reversible inhibitors of cysteine proteases, such as calpain, are peptide aldehydes, in particular dipeptide or tripeptide aldehydes, such as Z-Val-Phe-H (MDL 28170) (S. Mehdi, Trends in Biol.Sci. 1991, 16, 150-3).

It is the object of the present invention to provide novel α-keto carbonyl calpain inhibitors, which can also be inhibitors of cell damage by oxidative stress through free radicals, and which preferentially act in muscle cells in comparison with known calpain inhibitors

Summary of the Invention

The present invention relates to novel α-keto carbonyl calpain inhibitors of the formula (I) and their tautomeric and isomeric forms, and also, where appropriate, physiologically tolerated salts.

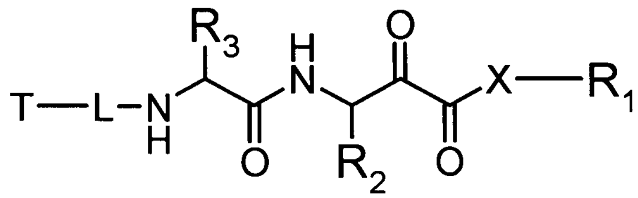

(I)

These α-keto carbonyl compounds are effective in the treatment of neurodegenerative diseases and neuromuscular diseases including Duchenne Muscular Dystrophy, Becker Muscular Dystrophy and other muscular dystrophies. Disuse atrophy and general muscle wasting can also be treated. Generally, all conditions where elevated levels of calpains are involved can be treated. The compounds of the invention may also inhibit other thiol proteases, such as cathepsin B, cathepsin H, cathepsin L and papain. Multicatalytic Protease (MCP) also known as proteasome may also be inhibited.

The compounds of the present invention are also inhibitors of cell damage by oxidative stress through free radicals and can be used to treat mitochondrial disorders and neurodegenerative diseases, where elevated levels of oxidative stress are involved.

The present invention also relates to pharmaceutical compositions comprising the compounds of the present invention and a pharmaceutically acceptable carrier.

Detailed Description of the Invention

The present invention relates to novel α-keto carbonyl calpain inhibitors of the formula (I) and their tautomeric and isomeric forms, and also, where appropriate, physiologically tolerated salts, where the variables have the following meanings:

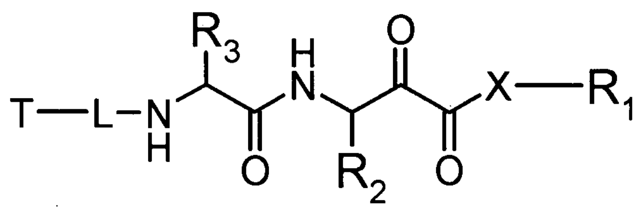

(I)

$R^1$ represents
- hydrogen,
- straight chain alkyl,
- branched chain alkyl,
- cycloalkyl,
- -alkylene-cycloalkyl,
- aryl,

-alkylene-aryl,
-$SO_2$-alkyl,
-$SO_2$-aryl,
-alkylene-$SO_2$-aryl,
-alkylene-$SO_2$-alkyl,
heterocyclyl or
-alkylene-heterocyclyl;

X represents O or NH;

$R^2$ represents
hydrogen,
straight chain alkyl,
branched chain alkyl,
cycloalkyl,
-alkylene-cycloalkyl,
aryl or
-alkylene-aryl;

$R^3$ represents
hydrogen,
straight chain alkyl,
branched chain alkyl,
cycloalkyl or
-alkylene-cycloalkyl;

L represents
a bond or at least one group selected from
-CO- ,
-CO-$(CH_2)_y$-CO- , wherein y is an integer of 1 to 6, i.e. 1, 2, 3, 4, 5 or 6,
-NH-$(CH_2)_z$-CO- , wherein z is an integer of 1 to 6, i.e. 1, 2, 3, 4, 5 or 6,
-CO-cycloalkylene-CO- ,
-NH-cycloalkylene-CO- ,
-CO-arylene-CO- and
-NH-arylene-CO-;

T is selected from the group consisting of

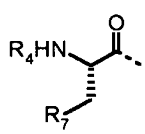
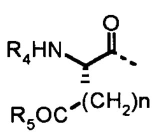
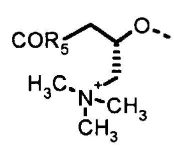
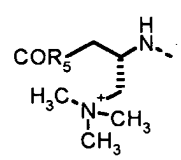
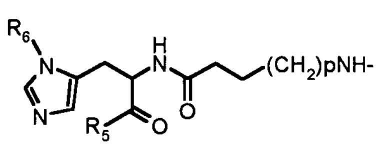
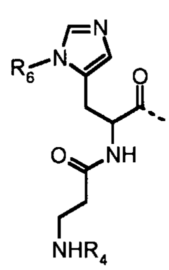
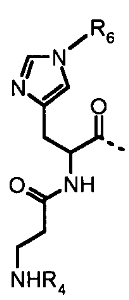
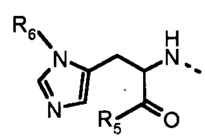
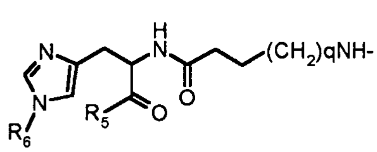
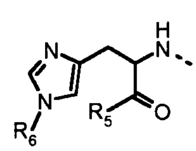

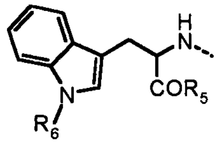
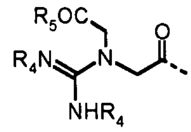
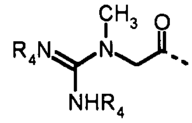
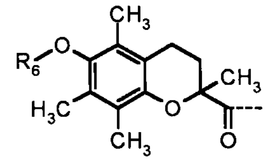

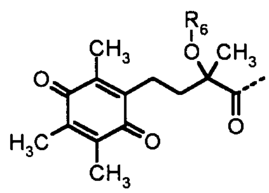
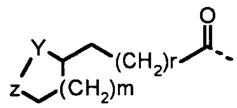
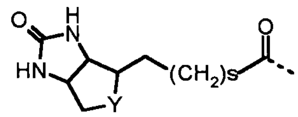
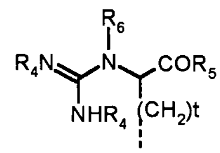

wherein each of m, n, p, q, r, s and t represents an integer of 0 to 6, i.e. 1, 2, 3, 4, 5 or 6;

$R^4$ represents

- hydrogen,
- $NH_2$-CO-,
- $NH_2$-CS-,
- $NH_2$-$SO_2$-,
- A-N H-CO-,
- $A_2$-N-CO-,
- A-NH-CS-,
- $A_2$-N-CS-,
- A-NH-$SO_2$-,
- $A_2$-N-$SO_2$-,
- A-CO-,
- A-CS-,
- A-$SO_2$-,
- A-O-CO- or
- A-O-CS-;

A is selected from the group consisting of

- straight chain or branched chain alkyl,
- straight chain or branched chain alkyl substituted with at least one halogen atom,
- straight chain or branched chain alkyl substituted with B,
- straight chain or branched chain alkyl substituted with at least one halogen atom and with B,
- cycloalkyl,
- cycloalkyl with at least one halogen atom,
- cycloalkyl substituted with B,
- cycloalkyl substituted with at least one halogen atom and with B,
- straight chain or branched chain alkyl with an attached cycloalkyl group,
- straight chain or branched chain alkyl with two attached cycloalkyl groups,
- straight chain or branched chain alkyl with an attached cycloalkyl group substituted with B,
- straight chain or branched chain alkyl with two attached cycloalkyl groups substituted with B,
- 1-adamantyl,
- 9-fluorenyl,
- phenyl,
- phenyl substituted with D,
- phenyl disubstituted with D,
- phenyl trisubstituted with D,
- naphthyl,
- naphthyl substituted with D,
- naphthyl disubstituted with D,

naphthyl trisubstituted with D,
straight chain or branched chain alkyl with an attached phenyl group,
straight chain or branched chain alkyl with two attached phenyl groups,
straight chain or branched chain alkyl with an attached phenyl group substituted with D,
straight chain or branched chain alkyl with two attached phenyl groups substituted with D,
straight chain or branched chain alkyl with an attached phenoxy group,
straight chain or branched chain alkyl with an attached phenoxy group substituted with D on the phenoxy group and
straight chain or branched chain alkyl with an attached 9-fluorenyl group;

B is selected from the group consisting of
halogen,
COOH,
OH,
CN,
$NO_2$,
$NH_2$,
alkoxy,
alkylamine,
dialkylamine,
alkyl-O-CO-,
alkyl-O-CO-NH- and
alkyl-S-;

D is selected from the group consisting of
halogen,
alkyl,
perfluoroalkyl,
alkoxy,
$NO_2$,
CN,
OH,
COOH,
$NH_2$,
alkylamino,
dialkylamino,
acyl,
alkyl-O-CO- and
alkyl-S-;

$R^5$ represents
XH,
$X^-$,
-X-alkyl,
-X-branched chain alkyl,
-X-cycloalkyl,
-X-alkylene-cycloalkyl,
-X-aryl,
-X-alkylene-aryl,
-X-$SO_2$-alkyl,
-X-$SO_2$-aryl,
-X-alkylene-$SO_2$-aryl,
-X-alkylene-$SO_2$-alkyl or
-X-alkylene-heterocyclyl,
wherein X is as defined above;

$R^6$ represents
hydrogen,

straight chain alkyl,
branched chain alkyl,
cycloalkyl,
-alkylene-cycloalkyl,
-alkylene-aryl,
A-O-CO-,
A-NH-CO-
A-CO -
A-$SO_2$- or
A-NH-$SO_2$-,
wherein A is as defined above;

$R^7$ represents
-$SO_3$H or
-$SO_2NHR_6$,
wherein $R^6$ has the meaning as defined above; and

Y and Z independently represents
S,
SO or
$CH_2$.

In the present invention, the substituents attached to formula (I) are defined as follows:

An alkyl group is a straight chain alkyl group, a branched chain alkyl group or a cycloalkyl group as defined below.

A straight chain alkyl group means a group $-(CH_2)_xCH_3$, wherein x is 0 or an integer of 1 or more. Preferably, x is 0 or an integer of 1 to 9, i.e. 1, 2, 3, 4, 5, 6, 7, 8 or 9, i.e the straight chain alkyl group has 1 to 10 carbon atoms. More preferred, x is 0 or an integer of 1 to 6, i.e. 1, 2, 3, 4, 5 or 6. Examples of the straight chain alkyl group are methyl, ethyl, n-propyl, n-butyl, n-pentyl, n-hexyl, n-heptyl, n-octyl, n-nonyl and n-decyl.

A branched chain alkyl group contains at least one secondary or tertiary carbon atom. For example, the branched chain alkyl group contains one, two or three secondary or tertiary carbon atoms. In the present invention, the branched chain alkyl group preferably has at least 3 carbon atoms, more preferably 3 to 10, i.e. 3, 4, 5, 6, 7, 8, 9 or 10, carbon atoms, further preferred 3 to 6 carbon atoms, i.e. 3, 4, 5 or 6 carbon atoms. Examples thereof are iso-propyl, sec.-butyl, tert.-butyl, 1,1-dimethyl propyl, 1,2-dimethyl propyl, 2,2-dimethyl propyl (neopentyl), 1,1-dimethyl butyl, 1,2-dimethyl butyl, 1,3-dimethyl butyl, 2,2-dimethyl butyl, 2,3-dimethyl butyl, 3,3-dimethyl butyl, 1-ethyl butyl, 2-ethyl butyl, 3-ethyl butyl, 1-n-propyl propyl, 2-n-propyl propyl, 1-iso-propyl propyl, 2-iso-propyl propyl, 1-methyl pentyl, 2-methyl pentyl, 3-methyl pentyl and 4-methyl pentyl.

In the present invention, a cycloalkyl group preferably has 3 to 8 carbon atoms, i.e. 3, 4, 5, 6, 7 or 8 carbon atoms. Examples thereof are cyclopropyl, cyclobutyl, cyclopentyl, cyclohexyl, cycloheptyl and cyclooctyl. More preferably, the cycloalkyl group has 3 to 6 carbon atoms, such as cyclopentyl, cyclohexyl and cycloheptyl.

In the present invention, the straight chain or branched chain alkyl group or cycloalkyl group may be substituted with at least one halogen atom selected from the group consisting of F, Cl, Br and I, among which F is preferred. Preferably, 1 to 5 hydrogen atoms of said straight chain or branched chain alkyl group or cycloalkyl group have been replaced by halogen atoms. Preferred haloalkyl groups include $-CF_3$, $-CH_2CF_3$ and $-CF_2CF_3$.

In the present invention, an alkoxy group is an -O-alkyl group, wherein alkyl is as defined above.

In the present invention, an alkylamino group is an -NH-alkyl group, wherein alkyl is as defined above.

In the present invention, a dialkylamino group is an $-N(alkyl)_2$ group, wherein alkyl is as defined above and the two alkyl groups may be the same or different.

In the present invention, an acyl group is a -CO-alkyl group, wherein alkyl is as defined above.

In an alkyl-O-CO- group, alkyl-O-CO-NH- group and alkyl-S- group, alkyl is as defined above.

An alkylene moiety may be a straight chain or branched chain group. Said alkylene moiety preferably has 1 to 6, i.e. 1, 2, 3, 4, 5 or 6, carbon atoms. Examples thereof include methylene, ethylene, n-propylene, n-butylene, n-pentylene, n-hexylene, methyl methylene, ethyl methylene, 1-methyl ethylene, 2-methyl ethylene, 1-ethyl ethylene, propyl methylene, 2-ethyl ethylene, 1-methyl propylene, 2-methyl propylene, 3-methyl propylene, 1-ethyl propylene, 2-ethyl propylene, 3-ethyl propylene, 1,1-dimethyl propylene, 1,2-dimethyl propylene, 2,2-dimethyl propylene, 1,1-dimethyl butylene, 1,2-dimethyl butylene, 1,3-dimethyl butylene, 2,2-dimethyl butylene, 2,3-dimethyl butylene, 3,3-dimethyl butylene, 1-ethyl butylene, 2-ethyl butylene, 3-ethyl butylene, 4-ethyl butylene, 1-n-propyl propylene, 2-n-propyl propylene, 1-iso-propyl propylene, 2-iso-propyl propylene, 1-methyl pentylene, 2-methyl pentylene, 3-methyl

pentylene, 4-methyl pentylene and 5-methyl pentylene. More preferably, said alkylene moiety has 1 to 4 carbon atoms, such as methylene, ethylene, n-propylene, 1-methyl ethylene and 2-methyl ethylene.

In the present invention, a cycloalkylene group preferably has 3 to 8 carbon atoms, i.e. 3, 4, 5, 6, 7 or 8 carbon atoms. Examples thereof are cyclopropylene, cyclobutylene, cyclopentylene, cyclohexylene, cycloheptylene and cyclooctylene. More preferably, the cycloalkylene group has 3 to 6 carbon atoms, such as cyclopropylene, cyclobutylene, cyclopentylene, and cyclohexylene. In the cycloalkylene group, the two bonding positions may be at the same or at adjacent carbon atoms or 1, 2 or 3 carbon atoms are between the two bonding positions. In preferred cycloalkylene groups the two bonding positions are at the same carbon atom or 1 or 2 carbon atoms are between the two bonding positions.

An aryl group is a carbocyclic or heterocyclic aromatic mono- or polycyclic moiety. The carbocyclic aromatic mono- or polycyclic moiety preferably has at least 6 carbon atoms, more preferably 6 to 20 carbon atoms. Examples thereof are phenyl, biphenyl, naphthyl, tetrahydronaphthyl, fluorenyl, indenyl and phenanthryl among which phenyl and naphthyl are preferred. Phenyl is especially preferred. The heterocyclic aromatic monocyclic moiety is preferably a 5- or 6-membered ring containing carbon atoms and at least one heteroatom, for example 1, 2 or 3 heteroatoms, such as N, O and/or S. Examples thereof are thienyl, pyridyl, furanyl, pyrrolyl, thiophenyl, thiazolyl and oxazolyl, among which thienyl and pyridyl are preferred. The heterocyclic aromatic polycyclic moiety is preferably an aromatic moiety having 6 to 20 carbon atoms with at least one heterocycle attached thereto. Examples thereof are benzothienyl, naphthothienyl, benzofuranyl, chromenyl, indolyl, isoindolyl, indazolyl, quinolyl, isoquinolyl, phthalazinyl, quinaxalinyl, cinnolinyl and quinazolinyl.

The aryl group may have 1, 2, 3, 4 or 5 substituents, which may be the same or different. Examples of said substituents are straight chain or branched chain alkyl groups as defined above, halogen atoms, such as F, Cl, Br or I, hydroxy groups, alkyloxy groups, wherein the alkyl moiety is as defined above, fluoroalkyl groups, i.e. alkyl groups as defined above, wherein 1 to (2x + 3) hydrogen atoms are substituted by fluoro atoms, especially trifluoro methyl, -COOH groups, -COO-alkyl groups and -CONH-alkyl groups, wherein the alkyl moiety is as defined above, nitro groups, and cyano groups.

An arylene group is a carbocyclic or heterocyclic aromatic mono- or polycyclic moiety attached to two groups of a molecule. In the monocyclic arylene group, the two bonding positions may be at adjacent carbon atoms or 1 or 2 carbon atoms are between the two bonding positions. In the preferred monocyclic arylene groups 1 or 2 carbon atoms are between the two bonding positions. In the polycyclic arylene group, the two bonding positions may be at the same ring or at different rings. Further, they may be at adjacent carbon atoms or 1 or more carbon atoms are between the two bonding positions. In the preferred polycyclic arylene groups 1 or more carbon atoms are between the two bonding positions. The carbocyclic aromatic mono- or polycyclic moiety preferably has at least 6 carbon atoms, more preferably 6 to 20 carbon atoms. Examples thereof are phenylene, biphenylene, naphthylene, tetrahydronaphthylene, fluorenylene, indenylene and phenanthrylene among which phenylene and naphthylene are preferred. Phenylene is especially preferred. The heterocyclic aromatic monocyclic moiety is preferably a 5- or 6-membered ring containing carbon atoms and at least one heteroatom, for example 1, 2 or 3 heteroatoms, such as N, O and/or S. Examples thereof are thienylene, pyridylene, furanylene, pyrrolylene, thiophenylene, thiazolylene and oxazolylene, among which thienylene and pyridylene are preferred. The heterocyclic aromatic polycyclic moiety is preferably an aromatic moiety having 6 to 20 carbon atoms with at least one heterocycle attached thereto. Examples thereof are benzothienylene, naphthothienylene, benzofuranylene, chromenylene, indolylene, isoindolylene, indazolylene, quinolylene, isoquinolylene, phthalazinylene, quinaxalinylene, cinnolinylene and quinazolinylene.

The arylene group may have 1, 2, 3, 4 or 5 substituents, which may be the same or different. Examples of said substituents are straight chain or branched chain alkyl groups as defined above, halogen atoms, such as F, Cl, Br or I, alkyloxy groups, wherein the alkyl moiety is as defined above, fluoroalkyl groups, i.e. alkyl groups a defined above, wherein 1 to (2x + 3) hydrogen atoms are substituted by fluoro atoms, especially trifluoro methyl.

The heterocyclyl group is a saturated or unsaturated non-aromatic ring containing carbon atoms and at least one hetero atom, for example 1, 2 or 3 heteroatoms, such as N, O and/or S. Examples thereof are morpholinyl, piperidinyl, piperazinyl, pyrrolidinyl and imidazolinyl.

In formula (I), $R^1$ may be a straight chain alkyl group as defined above. Preferably, x is 0 or an integer of 1 to 3, , i.e. the straight chain alkyl group of $R^1$ is preferably selected from methyl, ethyl, n-propyl and n-butyl. Especially preferred, the straight chain alkyl group is ethyl.

In formula (I), $R^1$ may be a branched chain alkyl group as defined above. More preferably, the branched chain alkyl group has 3 or 4 carbon atoms, examples thereof being iso-propyl, sec.-butyl and tert.-butyl.

In formula (I), $R^1$ may be a cycloalkyl group as defined above.

In formula (I), $R^1$ may be an -alkylene-cycloalkyl group. Therein, the alkylene moiety and the cycloalkyl group are as defined above.

In formula (I), $R^1$ may be an aryl group as defined above.

In formula (I), $R^1$ may be an -alkylene-aryl group. Therein, the alkylene moiety and the aryl group are as

defined above. More preferred, the alkylene moiety contains 1 to 4 carbon atoms. The more preferred aryl group attached to an alkylene moiety is mono- or bicyclic aryl.

In formula (I), $R^1$ may be an $SO_2$-alkyl group, wherein alkyl is as defined above.

In formula (I), $R^1$ may be an $SO_2$-aryl group, wherein aryl is as defined above.

In formula (I), $R^1$ may be an -alkylene-$SO_2$-aryl group, wherein alkylene and aryl are as defined above. More preferred, the alkylene moiety contains 1 to 4 carbon atoms. The more preferred aryl group attached to the $SO_2$-moiety is mono- or bicyclic aryl.

In formula (I), $R^1$ may be an -alkylene-$SO_2$-alkyl group, wherein alkylene and alkyl are as defined above. More preferred, the alkylene moiety contains 1 to 4 carbon atoms.

In formula (I), $R^1$ may be a heterocyclyl group as defined above.

In formula (I), $R^1$ may be an -alkylene-heterocyclyl group, wherein the alkylene moiety and the heterocyclyl group are as defined above.

Preferably, $R^1$ is selected from the group consisting of straight chain or branched chain alkyl, cycloalkyl, -alkylene-cycloalkyl, -alkylene-aryl, -alkylene-heterocyclyl and -alkylene-$SO_2$-aryl.

In formula (I), $R^2$ may be a straight chain alkyl group as defined above.

In formula (I), $R^2$ may be a branched chain alkyl group as defined above. More preferably, the branched chain alkyl group has 3 or 4 carbon atoms, examples thereof being iso-propyl, sec.-butyl and 1-methyl-propyl.

In formula (I), $R^2$ may be an aryl group as defined above.

In formula (I), $R^2$ may be an -alkylene-aryl group. Therein, the alkylene moiety and the aryl group are as defined above. More preferred, the alkylene moiety is methylene. More preferred, the alkylene moiety is a methylene group. The more preferred aryl group attached to the alkylene moiety is an optionally substituted phenyl group having one or two substituents. Preferred substituents are selected from the group consisting of halogen atoms, especially F and/or Cl and/or Br, alkyloxy groups, especially methoxy or ethoxy, fluoroalkyl groups, such as trifluoromethyl, and nitro and cyano groups.

Preferably, $R^2$ is a substituted or unsubstituted benzyl group.

In formula (I), $R^3$ may be a straight chain alkyl group as defined above.

In formula (I), $R^3$ may be a branched chain alkyl group as defined above. More preferably, the branched chain alkyl group has 3 or 4 carbon atoms, examples thereof being iso-propyl, sec.-butyl and 1-methyl-propyl.

In formula (I), $R^3$ may be a cycloalkyl group as defined above.

In formula (I), $R^3$ may be an -alkylene-cycloalkyl group. Therein, the alkylene moiety and the cycloalkyl group are as defined above.

Preferably, $R^3$ is a branched chain alkyl group, a cycloalkyl group, or an -alkylene-cycloalkyl group as defined above.

In formula (I), L may be $-CO-(CH_2)_y-CO-$ , wherein y is an integer of 1 to 6. Preferably, y is 2, 3 or 4.

In formula (I), L may be $-NH-(CH_2)_z-CO-$ , wherein z is an integer of 1 to 6. Preferably, z is 1, 2, 3 or 4.

In formula (I), L may be -CO-cycloalkylene-CO- or -NH-cycloalkylene-CO- as defined above.

In formula (I), L may be -CO-arylene-CO- or -NH-arylene-CO- as defined above.

In formula (I), L may be a combination of at least two groups, for example of two, three or four groups, selected from -CO- , $-CO-(CH_2)_y-CO-$ , wherein y is an integer of 1 to 6, i.e. 1, 2, 3, 4, 5 or 6, $-NH-(CH_2)_z-CO-$ , wherein z is an integer of 1 to 6, i.e. 1, 2, 3, 4, 5 or 6, -CO-cycloalkylene-CO-, -NH-cycloalkylene-CO- , CO-arylene-CO- and -NH-arylene-CO-. Preferably, L is a combination of two groups selected from $-CO-(CH_2)_y-CO-$ , wherein y is an integer of 1 to 6, and $-NH-(CH_2)_z-CO-$ ,

wherein z is an integer of 1 to 6. Therein, y is preferably 2, 3 or 4, especially preferred 2. z is preferably 2, 3, 4 or 5.

Preferably, L is a bond or a group selected from $-CO-(CH_2)_y-CO-$ , wherein y is an integer of 1 to 6, or -CO-cycloalkylene-CO-, $-NH-(CH_2)_z-CO-$ , wherein z is an integer of 1 to 6, or -NH-cycloalkylene-CO-, or a combination of two of these groups as defined above.

In the present invention, T is preferably selected from the group consisting of

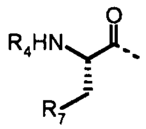
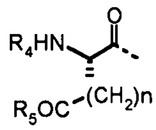
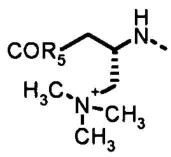
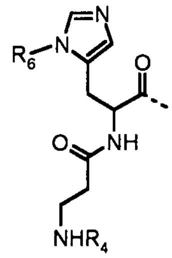
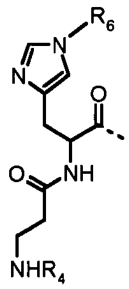
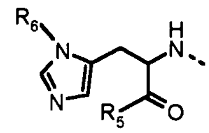
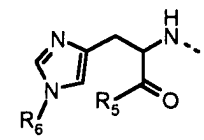

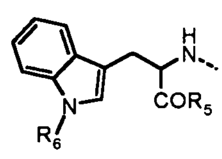
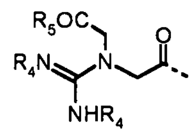
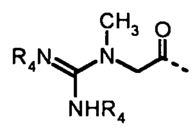
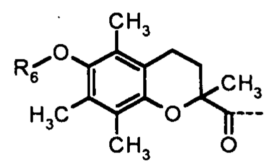

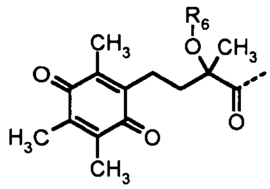
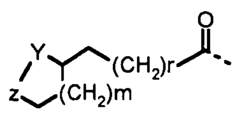
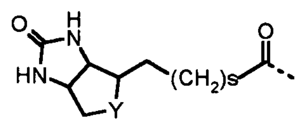
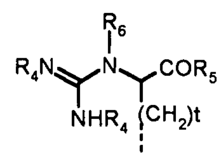

In one embodiment of the present invention, T is selected from the group consisting of

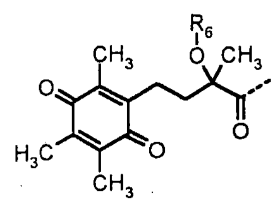
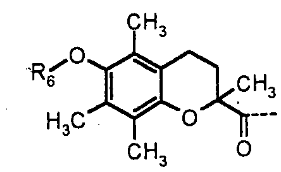

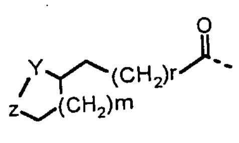
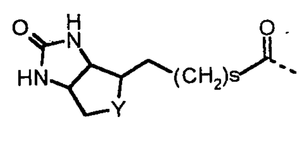

wherein $R^6$, m, r, s, Y and Z are as defined above. Especially preferred in this embodiment, Y and Z are S.

In each of groups T having a group $R^4$, $R^4$ is as defined above. Preferably, $R^4$ is selected from the group consisting of A-O-CO-, A-NH-CO-, A-CO-, and A-NH-$SO_2$-.

In the present invention, A may be a straight chain alkyl group, which has preferably 1 to 10 carbon atoms as defined above.

In the present invention, A may be a branched chain alkyl group, which has preferably 3 to 10 carbon atoms as defined above. More preferred, the branched chain alkyl group has 3 to 6 carbon atoms. Especially preferred, the branched chain alkyl group is tert.-butyl.

In the present invention, A may be a straight chain alkyl group, which has preferably 1 to 10 carbon atoms as defined above, and is substituted with at least one halogen atom selected from F, Cl, Br and I, among which F is preferred. Especially preferred haloalkyl groups include $-CF_3$, $-CH_2CF_3$ and $-CF_2CF_3$.

In the present invention, A may be a branched chain alkyl group, which has preferably 3 to 10 carbon atoms as defined above and is substituted with at least one halogen atom selected from F, Cl, Br and I, among which F is preferred. Especially preferred are perfluoroalkyl groups.

In the present invention, A may be a straight chain or branched chain alkyl group as defined above, which is substituted with B. Further, A may be a straight chain or branched chain alkyl group substituted with at least one halogen atom as defined above, which is in addition substituted with B as defined above.

In case B is selected from the group consisting of -O-alkyl, -NH-alkyl, -N(alkyl)$_2$, alkyl-O-CO-NH- and alkyl-S-, alkyl is preferably a straight chain or branched alkyl group having 1 to 10 carbon atoms. In the group -N(alkyl)$_2$, the alkyl groups independently preferably have 1 to 6 carbon atoms.

In the present invention, A may be a group as defined above, which is substituted with D as defined above. In case D is selected from the group consisting of alkyl, perfluoroalkyl, alkoxy, alkylamino, dialkylamino, acyl, alkyl-O-CO- and alkyl-S-, alkyl is as defined above.

Preferably, $R^4$ is A-O-CO-, A-CO-, A-SO$_2$- or A-NH-CO-, wherein A is alkyl, - alkylene-cycloalkyl, aryl or -alkylene-aryl, -alkylene-heterocyclyl as defined above. Especially, $R^4$ is selected from the group consisting of A-O-CO-, A-NH-CO-, A-CO-, and A-SO$_2$- wherein A is a straight chain alkyl group, which has 1 to 10 carbon atoms, a branched chain alkyl group, which has 3 to 10 carbon atoms, a cycloalkyl group, having 3 to 8 carbon atoms, an -alkylene-cycloalkyl group wherein the alkylene moiety is a straight chain alkylene group having 1 to 6 carbon atoms, and the cycloalkyl group has 3 to 8 carbon atoms, an aryl group, an -alkylene-aryl group, wherein the alkylene moiety is a straight chain alkylene group of 1 to 6 carbon atoms, and the aryl group is selected from substituted or unsubstituted phenyl, naphthyl, thienyl and pyridyl, or an -alkylene-heterocyclyl group wherein the alkylene moiety is a straight chain alkylene group of 1 to 6 carbon atoms.

In each of groups T having a group $R^5$, $R^5$ is as defined above. Preferably, $R^5$ is selected from the group consisting of XH; $X^-$; -X-(CH$_2$)$_x$CH$_3$ as defined above, wherein x is preferably an integer of 1 to 6; -X-branched chain alkyl having 3 to 6 carbon atoms as defined above; -X-cycloalkyl having 3 to 8 carbon atoms as defined above; -X-alkylene-cycloalkyl, wherein the alkylene moiety is a straight chain alkylene group preferably having 1 to 6 carbon atoms as defined above, and the cycloalkyl group has 3 to 8 carbon atoms as defined above; -X-aryl selected from substituted or unsubstituted phenyl, naphthyl, thienyl and pyridyl as defined above; -X-alkylene-aryl, wherein the alkylene moiety is a straight chain alkylene group preferably having 1 to 6 carbon atoms as defined above, and the aryl group is selected from substituted or unsubstituted phenyl, naphthyl, thienyl and pyridyl as defined above;-X-SO$_2$-alkyl, -X-SO$_2$-aryl, -X-alkylene-SO$_2$-aryl, -X-alkylene-SO$_2$-alkyl or -X-alkylene-heterocyclyl, wherein X is as defined above.

More preferably, $R^5$ is -X-alkyl, -X-branched chain alkyl, especially -o-tert.-butyl, - X-cycloalkyl, -X-alkylene-cycloalkyl, -X-aryl, -X-alkylene-aryl or -X-alkylene-heterocyclyl. Especially $R^5$ is -X-(CH$_2$)$_x$CH$_3$, wherein x is an integer of 1 to 6; -X-branched chain alkyl having 3 to 6 carbon atoms, -X-cycloalkyl having 3 to 8 carbon atoms, -X-alkylene-cycloalkyl, wherein the alkylene moiety is a straight chain alkylene group having 1 to 6 carbon atoms, and the cycloalkyl group has 3 to 8 carbon atoms; -X-aryl selected from substituted or unsubstituted phenyl, naphthyl, thienyl and pyridyl, -X-alkylene-aryl, wherein the alkylene moiety is a straight chain alkylene group having 1 to 6 carbon atoms, and the aryl group is selected from substituted or unsubstituted phenyl, naphthyl, thienyl and pyridyl, -X-alkylene-heterocyclyl wherein the alkylene moiety is a straight chain alkylene group of 1 to 6 carbon atoms, -X-cycloalkyl, -X-alkylene-cycloalkyl, -X-aryl, -X-alkylene-aryl or -X-alkylene-heterocyclyl.

In each groups T having a group $R^6$, $R^6$ is as defined above. Preferably, $R^6$ is hydrogen, a straight chain alkyl group having 1 to 6 carbon atoms as defined above, preferably 1 to 3 carbon atoms, especially being methyl; a branched chain alkyl group having 3 to 8 carbon atoms as defined above, preferably 3 to 6 carbon atoms, a cycloalkyl group having 3 to 8 carbon atoms as defined above; -alkylene-cycloalkyl, wherein the alkylene moiety is a straight chain alkylene group preferably having 1 to 6 carbon atoms as defined above, and the cycloalkyl group has 3 to 8 carbon atoms as defined above; -alkylene-aryl, wherein the alkylene moiety is a straight chain alkylene group preferably having 1 to 6 carbon atoms as defined above, and the aryl group is selected from substituted or unsubstituted phenyl, naphthyl, thienyl and pyridyl as defined above; A-O-CO-, A-NH-CO-, A-CO-, and A-NH-SO$_2$-, wherein A may be a straight chain alkyl group, which preferably has 1 to 10 carbon atoms as defined above, or a branched chain alkyl group, which preferably has 3 to 10 carbon atoms as defined above, or a cycloalkyl, -alkylene-cycloalkyl, aryl, or -alkylene-aryl group, or -alkylene-heterocyclyl as defined above. More preferably, the branched chain alkyl group has 3 to 6 carbon atoms. Especially preferred, the branched chain alkyl group is tert.-butyl.

In each groups T having a group $R^7$, $R^7$ is as defined above. Preferably, $R^7$ is -SO$_2$NHR$_6$.

Preferably, in the present invention, Y and Z independently represent S or SO. More preferably, Y and Z are both S or Y is S and Z is SO or Y is SO and Z is S.

The compounds of structural formula (I) are effective calpain inhibitors and may also inhibit other thiol proteases, such as cathepsin B, cathepsin H, cathepsin L or papain. Multicatalytic Protease (MCP) also known as proteasome may also be inhibited. The compounds of formula (I) are particularly effective as calpain inhibitors and are

therefore useful for the treatment and/or prevention of disorders responsive to the inhibition of calpain, such as neurodegenerative diseases and neuromuscular diseases including Duchenne Muscular Dystrophy, Becker Muscular Dystrophy and other muscular dystrophies, like disuse atrophy and general muscle wasting and other diseases with the involvement of calpain.

Optical Isomers - Diastereomers - Geometric Isomers - Tautomers

The compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Some of the compounds described herein may exist as tautomers such as ketoenol tautomers. The individual tautomers as well as mixtures thereof are encompassed within the compounds of structural formula (I).

The compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

Salts

The term "pharmaceutically acceptable salts" refers to salts prepared from pharmaceutically acceptable non-toxic bases or acids including inorganic or organic bases and inorganic or organic acids. Salts derived from inorganic bases include, for example, aluminum, ammonium, calcium, copper, ferric, ferrous, lithium, magnesium, manganic, manganous, potassium, sodium and zinc salts. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine and tromethamine.

When the compound of the present invention is basic, salts may be prepared from pharmaceutically acceptable non-toxic acids, including inorganic and organic acids. Such acids include, for example, acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, ptoluenesulfonic and trifluoroacetic acid. Particularly preferred are citric, fumaric, hydrobromic, hydrochloric, maleic, phosphoric, sulfuric and tartaric acid.

It will be understood that, as used herein, references to the compounds of formula (I) are meant to also include the pharmaceutically acceptable salts.

Utility

The compounds of formula (I) are calpain inhibitors and as such are useful for the preparation of a medicament for the treatment, control or prevention of diseases, disorders or conditions responsive to the inhibition of calpain such as neurodegenerative diseases and neuromuscular diseases including Duchenne Muscular Dystrophy, Becker Muscular Dystrophy and other muscular dystrophies. Neuromuscular diseases such as muscular dystrophies, include dystrophinopathies and sarcoglycanopathies, limb girdle muscular dystrophies, congenital muscular dystrophies, congenital myopathies, distal and other myopathies, myotonic syndromes, ion channel diseases, malignant hyperthermia, metabolic myopathies, hereditary cardiomyopathies, congenital myasthenic syndromes, spinal muscular atrophies, hereditary ataxias, hereditary motor and sensory neuropathies, hereditary paraplegias, and other neuromuscular disorders, as defined in *Neuromuscular Disorders* (2003), vol. 13. p:97-108. Disuse atrophy and general muscle wasting can also be treated. Generally all conditions where elevated levels of calpains are involved can be treated, including, for example, ischemias of the heart (eg. cardiac infarction), of the kidney or of the central nervous system (eg. stroke), inflammations, muscular dystrophies, cataracts of the eyes, injuries to the central nervous system (eg. trauma) and Alzheimer's disease. The compounds of formula (I) may also inhibit other thiol proteases such as, cathepsin B, cathepsin H, cathepsin L and papain. Multicatalytic Protease (MCP) also known as proteasome may also be inhibited by

the compounds of the invention.

The compounds of formula (I) are also inhibitors of cell damage by oxidative stress through free radicals and can be used to treat mitochondrial disorders and neurodegenerative diseases, where elevated levels of oxidative stress are involved.

Especially, those compounds of formula (I) wherein T is selected from

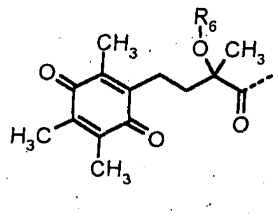

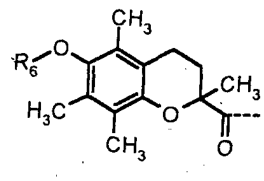

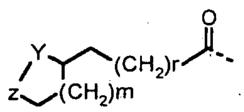

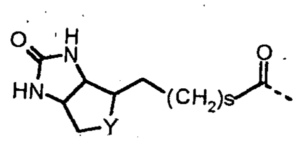

wherein $R^6$, m, r, s, Y and Z are as defined above are as defined above and act as inhibitors of cell damage by oxidative stress through free radicals and can be used to treat mitochondrial disorders and neurodegenerative diseases, where elevated levels of oxidative stress are involved. Among these compounds, those wherein Y and Z are S are especially preferred.

Mitochondrial disorders include Kearns-Sayre syndrome, mitochondrial encephalomyopathy-lactic-acidosis-stroke like episodes (MELAS), myoclonic epilepsy and ragged-red-fibers (MERRF), Leber hereditary optic neuropathy (LHON), Leigh's syndrome, neuropathy-ataxia-retinitis pigmentosa (NARP) and progressive external opthalmoplegia (PEO) summarized in Schapira and Griggs (eds) 1999 *Muscle Diseases,* Butterworth-Heinemann.

Neurodegenerative diseases with free radical involvement include degenerative ataxias, such as Friedreich' Ataxia, Parkinson's disease, Huntington's disease, amyotrophic lateral sclerosis (ALS) and Alzheimer's disease (Beal MF, Howell N., Bodis-Wollner I (eds), 1997, *Mitochondria and free radicals in neurodegenerative diseases,* Wiley-Liss).

## Administration and Dose Ranges

Any suitable route of administration may be employed for providing a mammal, especially a human, with an effective dosage of a compound of the present invention. For example, oral, rectal, topical, parenteral, ocular, pulmonary or nasal administration may be employed. Dosage forms include, for example, tablets, troches, dispersions, suspensions, solutions, capsules, creams, ointments and aerosols. Preferably the compounds of formula (I) are administered orally, parenterally or topically.

The effective dosage of the active ingredient employed may vary depending on the particular compound employed, the mode of administration, the condition being treated and the severity of the condition being treated. Such dosage may be ascertained readily by a person skilled in the art.

When treating Duchenne Muscular Dystrophy, Becker Muscular Dystrophy and other muscular dystrophies, generally, satisfactory results are obtained when the compounds of the present invention are administered at a daily dosage of about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating ischemias of the heart (eg. cardiac infarction), of the kidney or of the central nervous system (eg. stroke), generally, satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating mitochondrial disorders or neurodegenerative diseases where oxidative stress is a factor, generally, satisfactory results are obtained when the compounds of the present invention are administered at a daily dosage

of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

Formulation

The compound of formula (I) is preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents and/or flavoring agents. The compositions of the invention may be formulated so as to provide quick, sustained or delayed release of the active ingredient after administration to the patient.

Preparation of Compounds of the Invention

The compounds of formula (I) of the present invention can be prepared according to the procedures of the following Schemes and Examples, using appropriate materials and are further exemplified by the following specific examples. Moreover, by utilizing the procedures described herein in conjunction with ordinary skills in the art additional compounds of the present invention can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously hereinabove. The free amine bases corresponding to the isolated salts can be generated by neutralization with a suitable base, such as aqueous sodium hydrogencarbonate, sodium carbonate, sodium hydroxide, and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation, or crystallization. All temperatures are degrees Celsius.

When describing the preparation of the present compounds of formula (I), the terms "T moiety", "L moiety" and "Dipeptide moiety" are used below. This moiety concept is illustrated below:

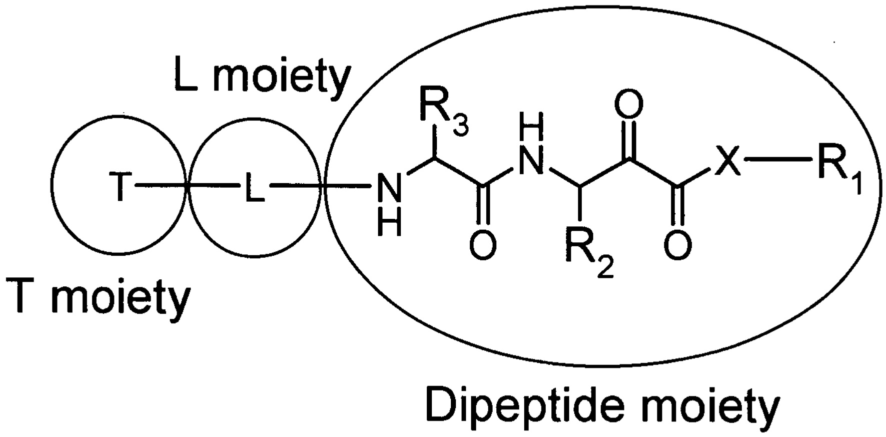

The preparation of the compounds of the present invention may be advantageously carried out via sequential synthetic routes. The skilled artisan will recognize that in general, the three moieties of a compound of formula (I) are connected via amide bonds. The skilled artisan can, therefore, readily envision numerous routes and methods of connecting the three moieties via standard peptide coupling reaction conditions.

The phrase "standard peptide coupling reaction conditions" means coupling a carboxylic acid with an amine using an acid activating agent such as EDC, dicyclohexylcarbodiimide, and benzotriazol-1-yl-N,N,N',N'-tetramethyluronium hexafluorophosphate in a inert solvent such as DMF in the presence of a catalyst such as HOBt. The uses of protective groups for amine and carboxylic acids to facilitate the desired reaction and minimize undesired reactions are well documented. Conditions required to remove protecting groups which may be present can be found in Greene, et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide such as palladium on activated carbon in a protic solvent such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas. Fmoc protecting groups can be removed with piperidine in a suitable solvent such as DMF.

The required dipeptide moieties can advantageously be prepared via a Passerini reaction (T. D. Owens et al., Tet. Lett., 42, 6271, (2001); L. Banfi et al., Tet. Lett., 43, 4067, (2002) from an $R^1$-isonitrile, a suitably protected $R^2$-aminoaldehyde, and a suitably protected $R^3$-amino acid followed by N-deprotection and acyl-migration, which leads to the corresponding dipeptidyl $\alpha$-hydroxy-amide. The groups $R^1$, $R^2$ and $R^3$ are as defined above with respect to formula (I). The reactions are carried out in an inert solvent such as $CH_2Cl_2$ at room temperature. The $\alpha$-keto amide functionality on the dipeptide moiety is typically installed using a Dess-Martin oxidation (S. Chatterjee et al., J. Med. Chem., 40, 3820, (1997)) in an inert solvent such as $CH_2Cl_2$ at 0 °C or room temperature. This oxidation can be carried out either following the complete assembly of the compounds of Formula (I) using peptide coupling reactions or at any convenient intermediate stage in the sequence of connecting the three moieties T, L, and dipeptide, as it will be readily recognized by those skilled in the art.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

In the above description and in the schemes, preparations and examples below, the various reagent symbols and abbreviations have the following meanings:

| | |
|---|---|
| Boc | t-butoxycarbonyl |
| DIEA | diisopropylethylamine |
| DMAP | 4-dimethylaminopyridine |
| DMF | N,N-dimethylformamide |
| EDC | 1-(3-dimethylaminopropyl)-3-ethylcarbodiimide hydrochloride |

| | |
|---|---|
| Et | ethyl |
| EtOAc | ethyl acetate |
| Fmoc | 9-fluorenylmethyl-carbamate |
| HBTU | benzotriazol-1-yl-N,N,N',N'-tetramethyluronium hexafluorophosphate |
| HOAc | acetic acid |
| HOAt | 1 -hydroxy-7-azabenzotriazote |
| HOBt | 1-hydroxybenzotriazole |
| h | hour(s) |
| NMM | N-methylmorpholine |
| Me | methyl |
| Phe | phenylalanine |
| PyBOP | benzotriazol-1-yloxytris(pyrrolidino)-phosphonium hexafluorophosphate |
| TFA | trifluoroacetic acid |
| TEA | triethylamine |
| Z | benzyloxycarbonyl |

Reaction Scheme 1: Coupling techniques for compounds of formula (I)

Technique 1

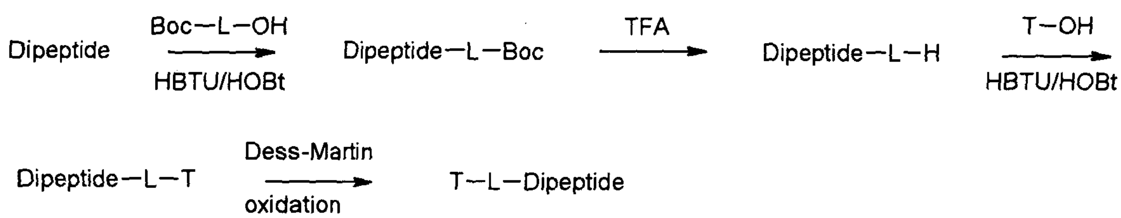


Technique 2

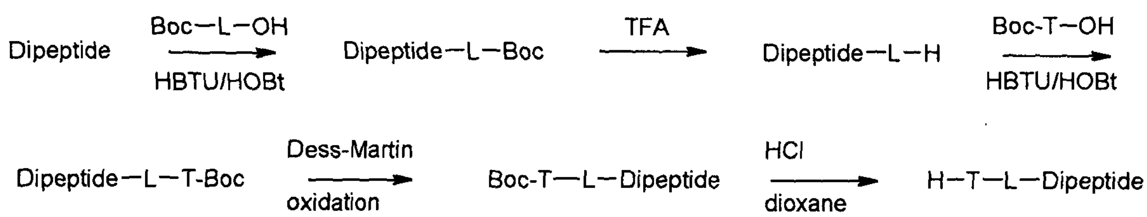


Technique 3

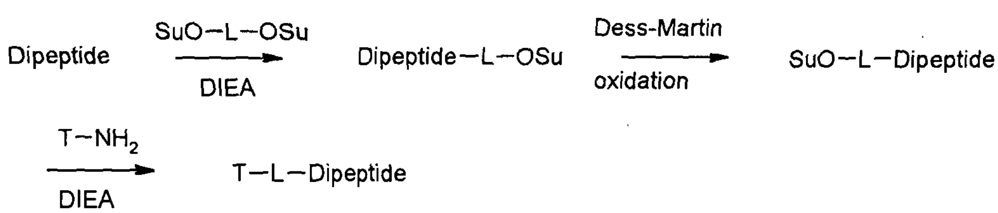


In coupling technique 1, an appropriate dipeptide moiety (e.g. $H_2N$-Val-Phe(4-Cl)-hydroxy-ethylamide) is coupled to an L moiety (e.g., Boc-Gly-OH) in the presence of HBTU/HOBt followed by Boc deprotection. The coupled L-dipeptide hydroxy-ethylamide compound is then coupled to an appropriate T moiety followed by Dess-Martin oxidation to the corresponding α-keto amide compound.

In coupling technique 2, an appropriate dipeptide moiety (e.g. $H_2N$-Val-Phe(4-Cl)-hydroxy-ethylamide) is coupled to an L moiety (e.g., Boc-Gly-OH) in the presence of HBTU/HOBt followed by Boc deprotection. The coupled L-dipeptide hydroxy-ethylamide compound is then coupled to an appropriately protected T moiety followed by Dess-Martin oxidation to the corresponding α-keto amide compound. Finally deprotection of the protecting group and salt formation is carried out.

In coupling technique 3, an appropriate dipeptide moiety (e.g. $H_2N$-Val-Phe(4-Cl)-hydroxy-ethylamide) is coupled to an L moiety in the form of its bis hydroxysuccinimidyl ester (e.g., $SuOCOCH_2CH_2COOSu$) in the presence of DIEA followed by Dess-Martin oxidation to the corresponding α-keto amide compound. The resulting mono hydroxysuccinimidyl ester is then coupled with to an free amine T moiety in the presence of DIEA and a suitable solvent typically DMF, $H_2O$ or a mixture of the two.

Generally, after a peptide coupling reaction is completed, the reaction mixture can be diluted with an appropriate organic solvent, such as EtOAc, $CH_2Cl_2$ or $Et_2O$, which is then washed with aqueous solutions, such as water, HCl, $NaHSO_4$, bicarbonate, $NaH_2PO_4$, phosphate buffer (pH 7), brine or any combination thereof The reaction mixture can be concentrated and then be partitioned between an appropriate organic solvent and an aqueous solution. The reaction mixture can be concentrated and subjected to chromatography without aqueous workup.

Protecting groups such as Boc, Z, Fmoc and $CF_3CO$ can be deprotected in the presence of $H_2$/Pd-C, TFA/DCM, HCl/EtOAc, HCl/doxane, HCl in MeOH/$Et_2O$, $NH_3$/MeOH or TBAF with or without a cation scavenger, such as thioanisole, ethane thiol and dimethyl sulfide (DMS). The deprotected amines can be used as the resulting salt or are freebased by dissolving in DCM and washing with aqueous bicarbonate or aqueous NaOH. The deprotected amines can also be freebased by ion exchange chromatography.

More detailed procedures for the assembly of compounds of formula (I) are described in the section with the examples of the present invention.

Reaction Scheme 2: Preparation of "Dipeptide moiety" employing the Passerini reaction

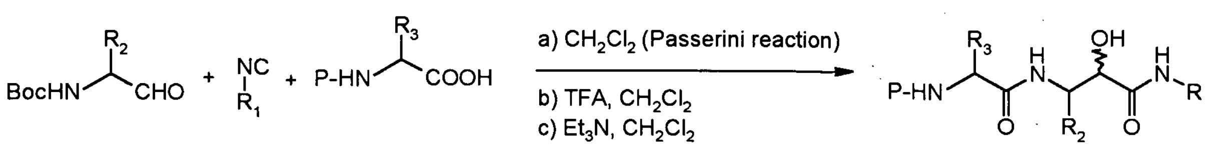


**1** **2** **3** **4**

P is an amino protecting group as described before; and $R^1$ to $R^3$ are as defined above with respect to formula (I).

The dipeptide moieties of the present invention, in general, may be prepared from commercially available starting materials via known chemical transformations. The preparation of a dipeptide moiety of the compound of the present invention is illustrated in the reaction scheme above.

As shown in Reaction Scheme 2, the "dipeptide moiety" of the compounds of the present invention can be prepared by a three-component reaction between a Boc-protected amino aldehyde **1**, an isonitrile **2** and a suitably protected amino acid **3** (Passerini reaction) in an organic solvent, such as $CH_2Cl_2$, at a suitable temperature. Following deprotection of the Boc group using TFA in a suitable solvent, such as $CH_2Cl_2$, the dipeptide moieties **4** are obtained after base-induced acyl-migration using a suitable base, such as $Et_3N$ or DIEA, in a suitable solvent, such as $CH_2Cl_2$. More detailed examples of dipeptide moiety preparation are described below.

Suitably functionalized L moieties are commercially available or can readily be prepared by the skilled artisan following published procedures (M. Hill et al., FEBS Lett., 102, 282, (1979)).

Suitably functionalized T moieties are commercially available or can readily prepared by the skilled artisan from commercial precursors by standard protecting group manipulations.

Synthesis Scheme for Example 1

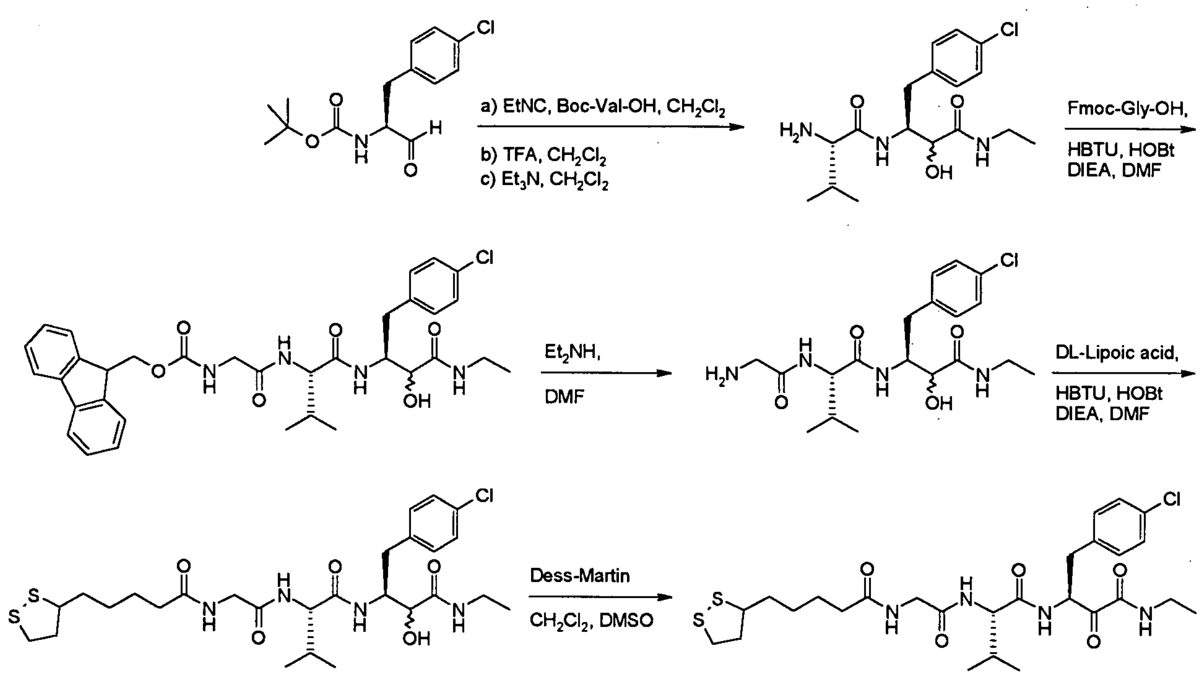


Synthesis Scheme for Example 26

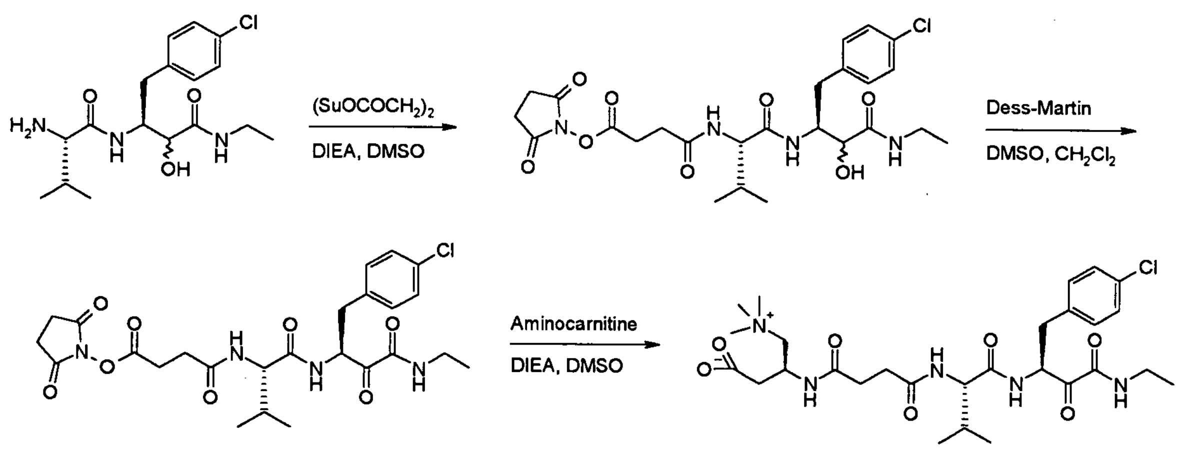

Synthesis Scheme for Example 39

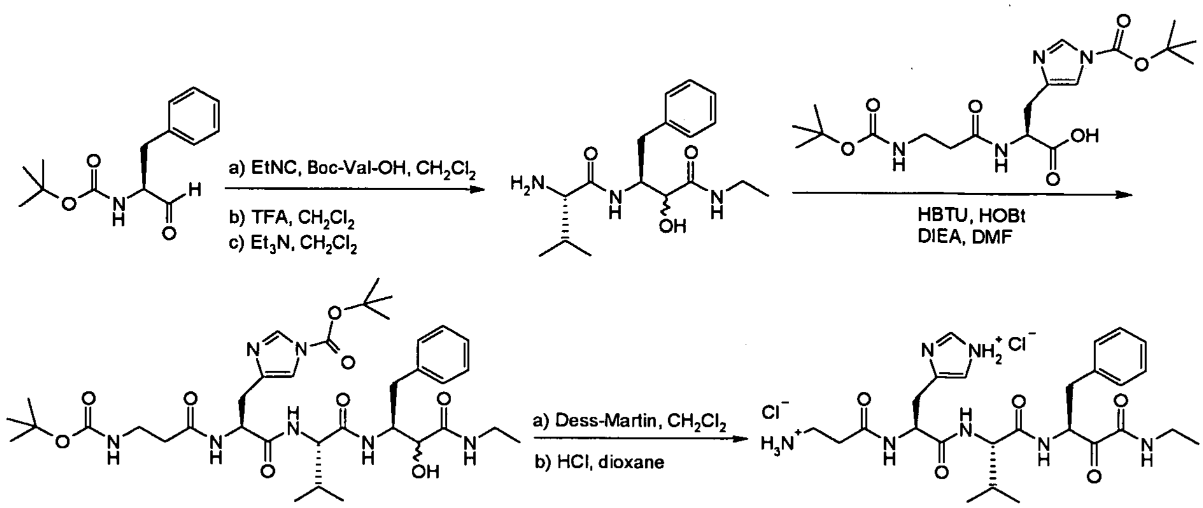


Synthesis Scheme for Example 59

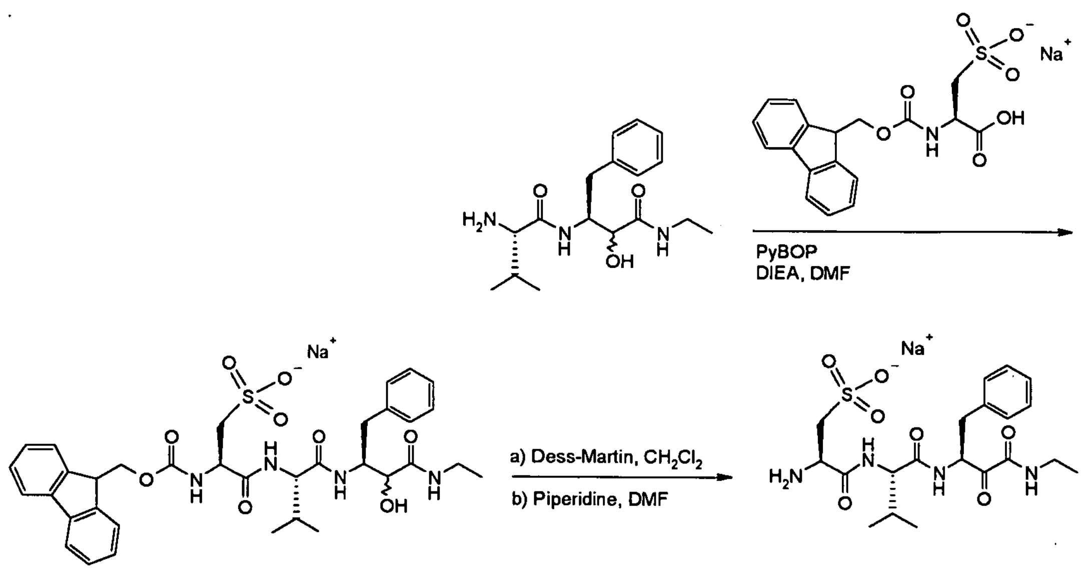


Example 1:

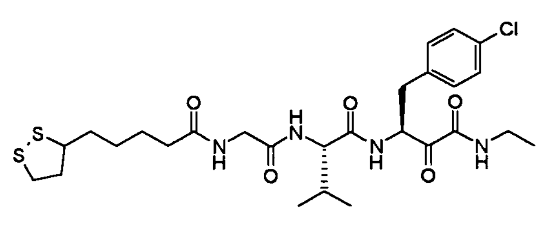

A solution of 12.0 mg of intermediate 1d) in 1.2 ml of DMSO and 1.2 ml of $CH_2Cl_2$ was cooled in ice. 10.2 mg of Dess-Martin reagent was added and the mixture was stirred in an ice bath for 60 min. The cooling bath was removed and stirring continued at r.t. for a further 60 min. $CH_2Cl_2$ was added and the mixture was washed 1 M $Na_2S_2O_3$, sat. $NaHCO_3$, and $H_2O$, dried with anh. $Na_2SO_4$ and evaporated in vacuo. The crude product was purified by column chromatography ($CH_2Cl_2$/MeOH 98:2 → $CH_2Cl_2$/MeOH 95:5) and dried in vacuo. Example 1 was obtained in form of a yellowish solid.
$R_f$ = 0.36 ($CH_2Cl_2$/MeOH 9:1); Mp. 198-200 °C.

The required intermediates can be synthesized in the following way:

*Intermediate 1a):*

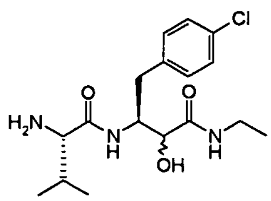

To a solution of 1.00 g of Boc-*p*-chloro-phenylalaninal in 14 ml of anh. $CH_2Cl_2$ was added 0.39 ml of Ethyl isocyanide, followed by 0.76 g of Boc-valine, and the mixture was stirred at r.t. for 18h. The resulting solution was evaporated to dryness and the residue redissolved in 14 ml of $CH_2Cl_2$. 5 ml of TFA was added and the reaction was stirred at r.t. for 2h. The volatiles were evaporated in vacuo and the residue dried in vacuo. The resulting yellow oil was dissolved in 14 ml of $CH_2Cl_2$, 10 ml of $Et_3N$ was added and the reaction was stirred at r.t. overnight. Then the reaction mixture was evaporated to dryness in vacuo and the residue was partitioned between 1N NaOH and EtOAc. The organic layer was washed with 1 N NaOH, $H_2O$, and brine. The aqueous layers were back extracted with EtOAc and the combined organic layer dried over $Na_2SO_4$ and evaporated in vacuo. The crude product was suspended in $Et_2O$, filtered off, washed with cold $Et_2O$, and dried in vacuo to yield intermediate 1a) as a white solid. $R_f$ = 0.27 ($CH_2Cl_2$/MeOH 9:1); Mp. 187-190 °C.

*Intermediate 1b):*

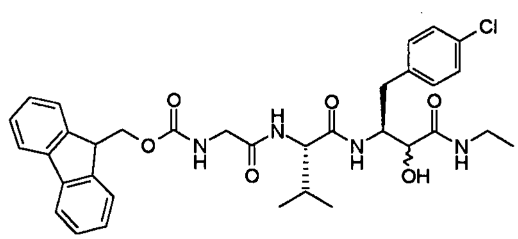

To a solution of 44.6 mg of Fmoc-Glycine and 27.0 mg of HOBt in 1.50 ml of DMF was added 56.9 mg of HBTU followed by 0.11 ml of DIEA and the mixture was stirred at r.t for 10 min. Then, 44.5 mg of intermediate 1a) was added and the reaction was stirred at r.t. overnight. The resulting solution was diluted with EtOAc, washed with 1N HCl (3x), sat $NaHCO_3$ (3x), $H_2O$, and brine. The organic layer was dried with anh. $MgSO_4$ and evaporated in vacuo. The crude product was suspended in $Et_2O$, filtered off, washed with cold $Et_2O$, and dried in vacuo to yield intermediate 1b) as a yellowish solid.
$R_f$ = 0.32 ($CH_2Cl_2$/MeOH 9:1); Mp. 175-177 °C.

*Intermediate 1c):*

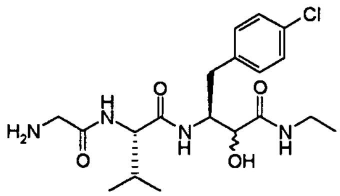

64 mg of Intermediate 1b) was dissolved in 2 ml of DMF and 0.18 ml of $Et_2NH$ was added. The reaction mixture was stirred at r.t. for 4h. The solution was evaporated in vacuo and the residue was suspended in $Et_2O$ and EtOAc, filtered off, and washed with cold $Et_2O$ to furnish intermediate 1c) as a yellowish solid which was dried in vacuo.

$R_f$ = 0.02 ($CH_2Cl_2$/MeOH 9:1); Mp. 223-225 °C.

*Intermediate 1d):*

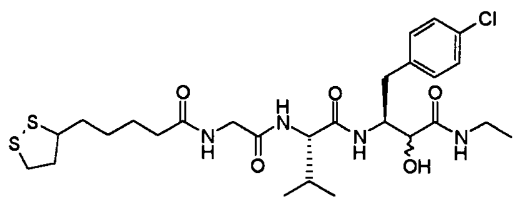

To a solution of 30.9 mg of DL-Lipoic acid and 27.0 mg of HOBt in 1.50 ml of DMF was added 56.9 mg of HBTU, followed by 0.11 ml of DIEA, and the mixture was stirred at r.t for 10 min. Then, 41.9 mg of intermediate 1c) was added and the reaction stirred at r.t. overnight. The resulting solution was diluted with EtOAc, washed with 1N HCl (3x), 2N $K_2CO_3$ (3x), $H_2O$, and brine. The organic layer was dried with anh. $MgSO_4$ and evaporated in vacuo. The crude product was triturated with hot $CH_2Cl_2$, filtered off, washed with cold $CH_2Cl_2$, and dried in vacuo to yield intermediate 1d) as a white solid.
$R_f$ = 0.22 ($CH_2Cl_2$/MeOH 9:1); Mp. 178-180 °C.
The compounds of the following examples can be prepared in a similar way:

<u>Example 2:</u>

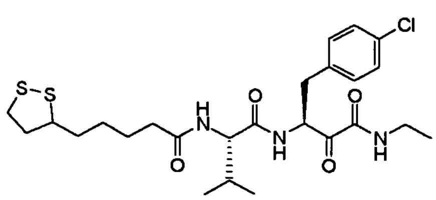

$R_f$ = 0.61 ($CH_2Cl_2$/MeOH 9:1); Mp. 209-212 °C.

Example 3:

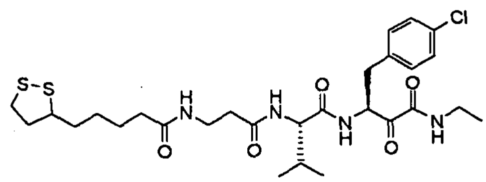

$R_f$=0.41 ($CH_2Cl_2$/MeOH 9:1).

Example 4:

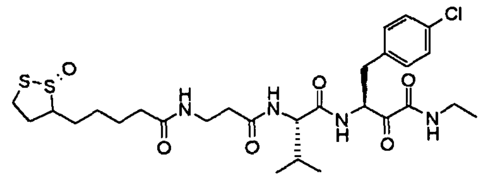

$R_f$ = 0.37 ($CH_2Cl_2$/MeOH 9:1).

Example 5:

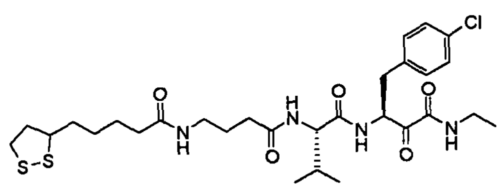

$R_f$ = 0.50 ($CH_2Cl_2$/MeOH 9:1).

Example 6:

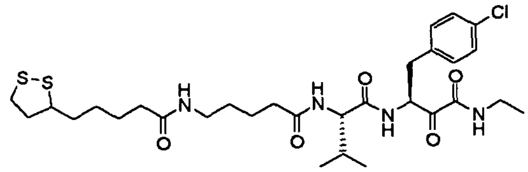

$R_f$ = 0.35 ($CH_2Cl_2$/MeOH 9:1); Mp. 219-220 °C.

Example 7:

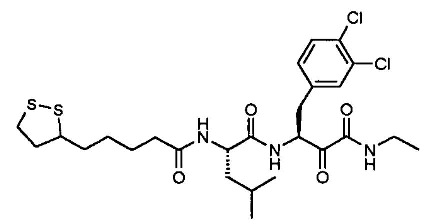

$R_f$ = 0.75 ($CH_2Cl_2$/MeOH 15:1).

Example 8:

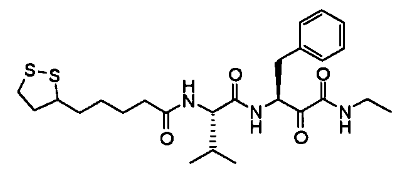

$R_f$ = 0.65 ($CH_2Cl_2$/MeOH 60:5).

Example 9:

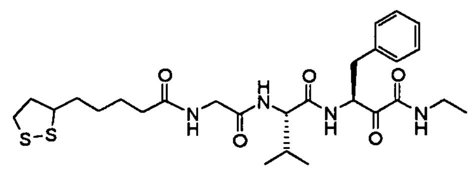

$R_f$ = 0.48 ($CH_2Cl_2$/MeOH 10:1).

Example 10:

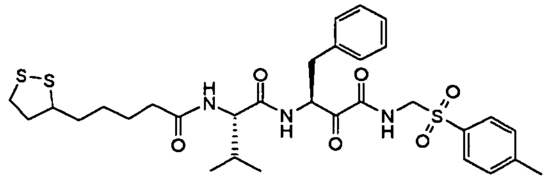

$R_f$ = 0.39 ($CH_2Cl_2$/MeOH 20:1), mp: 180 °C.

Example 11:

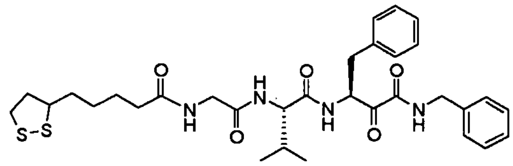

$R_f$ = 0.40 ($CH_2Cl_2$/MeOH 10:1).

Example 12:

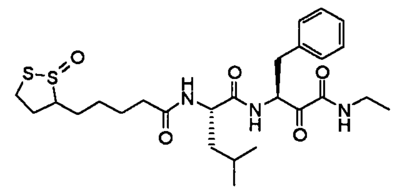

$R_f$ = 0.31 ($CH_2Cl_2$/MeOH 95:5).

Example 13:

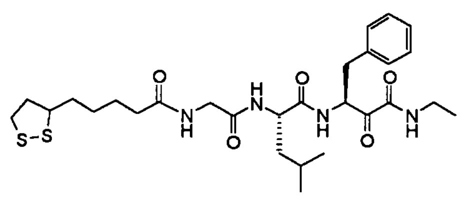

$R_f$ = 0.44 ($CH_2Cl_2$/MeOH 95:5).

Example 14:

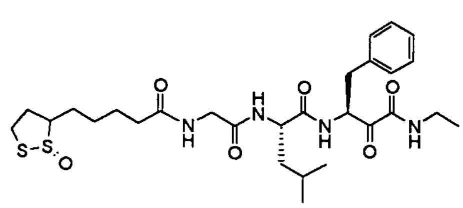

$R_f$ = 0.22 ($CH_2Cl_2$/MeOH 95:5).

Example 15:

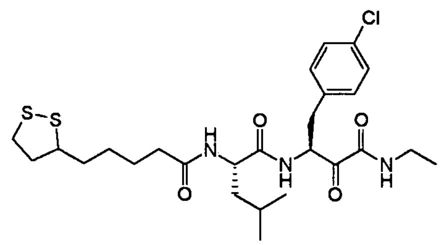

$R_f$ = 0.20 ($CH_2Cl_2$/MeOH 95:5); Mp.144-150 °C.

Example 16:

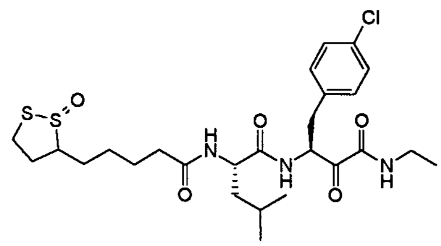

$R_f$ = 0.15 ($CH_2Cl_2$/MeOH 95:5); Mp. 154-155 °C.

Example 17:

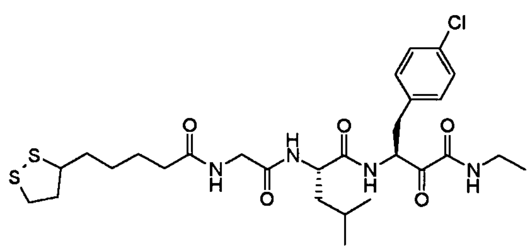

$R_f$ = 0.17 ($CH_2Cl_2$/MeOH 95:5); Mp. 144-147 °C.

Example 18:

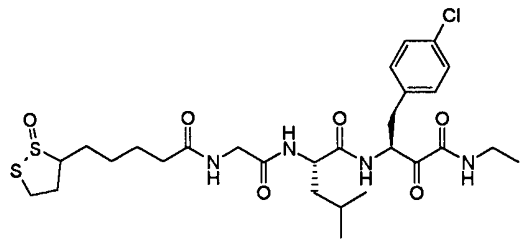

$R_f$ = 0.11 ($CH_2Cl_2$/MeOH 95:5); Mp.124-127 °C.

Example 19:

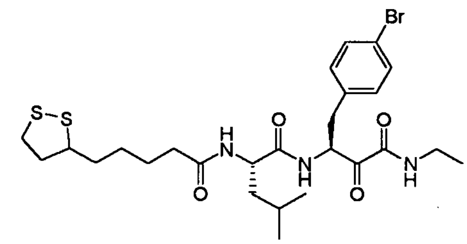

$R_f$ = 0.44 ($CH_2Cl_2$/MeOH 95:5); Mp. 155-158 °C.

Example 20:

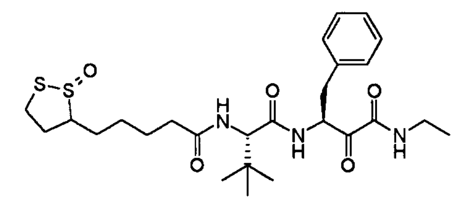

$R_f$ = 0.37 ($CH_2Cl_2$/MeOH 9:1).

Example 21:

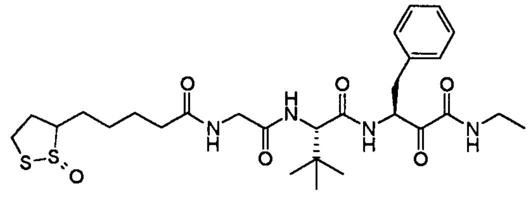

$R_f$ = 0.55 ($CH_2Cl_2$/MeOH 9:1); Mp. 181-182 °C

Example 22:

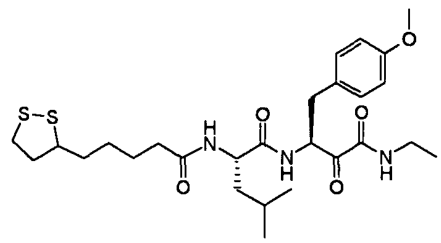

$R_f$ = 0.65 ($CH_2Cl_2$/MeOH 9:1); Mp. 120-121 °C.

Example 23:

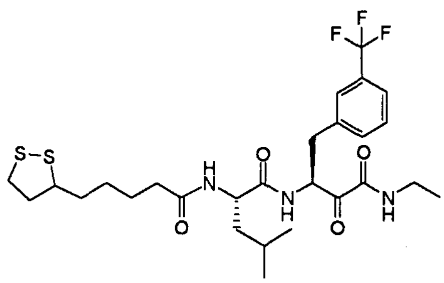

$R_f$ = 0.57 ($CH_2Cl_2$/MeOH 9:1).

Example 24:

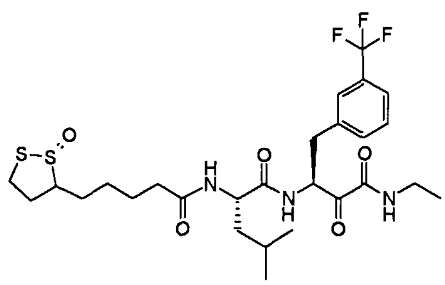

$R_f$ = 0.44 ($CH_2Cl_2$/MeOH 9:1); Mp. 160-162 °C.

Example 25:

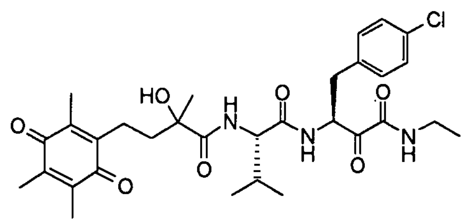

$R_f$ = 0.59 ($CH_2Cl_2$/MeOH 10:1).

Example 26:

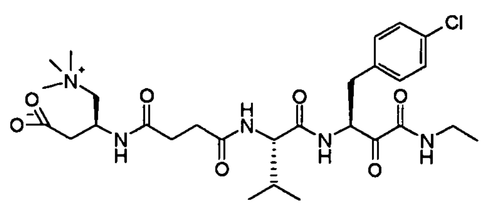

To a solution of 27.7 mg of intermediate 26b) in 2 ml DMF was added a solution of 6.4 mg of Aminocarnitine in 0.3 ml $H_2O$, followed by 34.8 ❑ DIEA. The reaction mixture was stirred at r.t. for 6 h and then evaporated in vacuo. The residue was dissolved in $H_2O$ (10 ml) and the solution washed with $CH_2Cl_2$ (3×10 ml). The aqueous phase was evaporated in vacuo. The crude product was redissolved in $H_2O$ and purified by reverse phase column chromatography on a C18-phase ($H_2O \rightarrow H_2O$/MeCN 1:1). After drying in vacuo over $P_2O_5$, example 26 was obtained as a colorless glassy solid.
$R_f$= 0.18 (MeOH/AcOH 9:1); 185-190 °C.

The required intermediates can be synthesized in the following way:

*Intermediate 26a):*

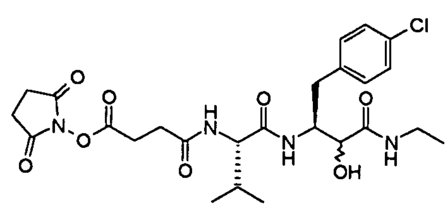

To a solution of 195.2 mg of bis-hydroxysuccinimide succinate in 5 ml of DMSO was added 44.5 mg of intermediate 1a) followed by 0.04 ml DIEA. The reaction mixture was stirred at r.t. for 3 d and evaporated in vacuo. The remaining DMSO was coevaporated with DMF (5×50 ml), the residue was resuspended in EtOAc (50 ml) and washed with $H_2O$ (3×25 ml). After drying with anh. $MgSO_4$ the crude product was dissolved in $CH_2Cl_2$/MeOH 1:1, adsorbed onto silica gel, and purified by column chromatography (EtOAc → EtOAc/MeOH 98:2). The material was further purified by a second column chromatography ($CH_2Cl_2$/MeOH 98:2 → $CH_2Cl_2$/MeOH 95:5) and dried in vacuo to yield intermediate 26a) as a white solid.
$R_f$ = 0.54 (EtOAc/MeOH 95:5).

*Intermediate 26b):*

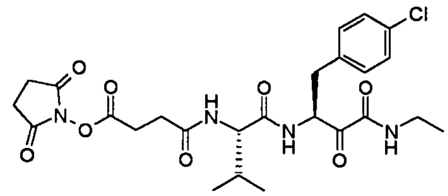

A solution of 138.3 mg of intermediate 26a) in 2 ml of DMSO and 10 ml of $CH_2Cl_2$ was cooled in ice. 106.0 mg of Dess-Martin reagent was added and the mixture stirred in the ice bath for 15 min. The cooling bath was removed and stirring continued at r.t. for 4 h. The reaction mixture was filtered. The filtrate was evaporated in vacuo and the remaining DMSO was coevaporated with DMF (5×15 ml). The crude product was redissolved in DMF (15 ml) and filtered again. The filtrate was evaporated to dryness in vacuo and the residue was triturated with hot EtOAc (10 ml). The insoluble material was filtered off, washed with EtOAc and $Et_2O$ and finally dried in vacuo. In this way intermediate 26b) was obtained as a white powder.
$R_f$ = 0.38 ($CH_2Cl_2$/MeOH 10:1).
The following examples can be prepared in a similar way:

Example 27:

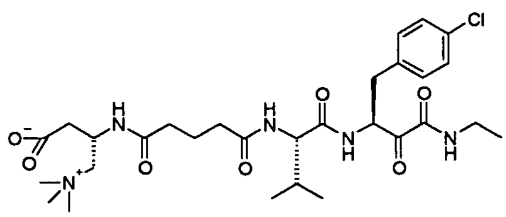

$R_f$ = 0.17 (MeOH/AcOH 9:1 ).

Example 28:

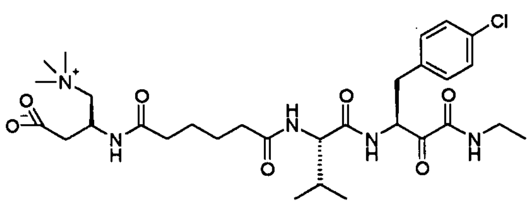

$R_f$ = 0.18 (MeOH/AcOH 9:1).

Example 29:

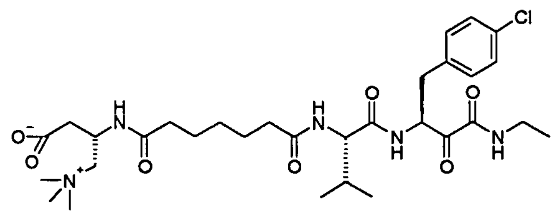

$R_f$ = 0.17 (MeOH/AcOH 9:1); Mp. 172-174 °C.

Example 30:

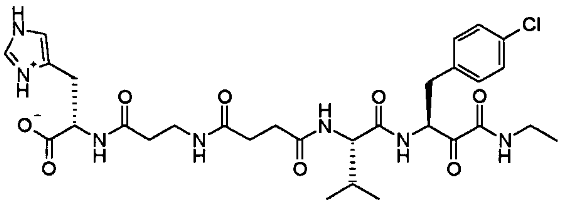

$R_f$ = 0.41 (MeOH/AcOH 9:1); Mp. 186-188 °C.

Example 31:

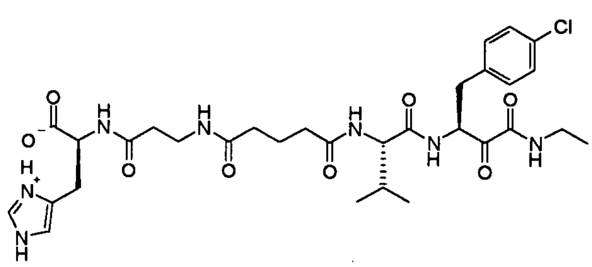

$R_f$ = 0.42 (MeOH/AcOH 9:1).

Example 32:

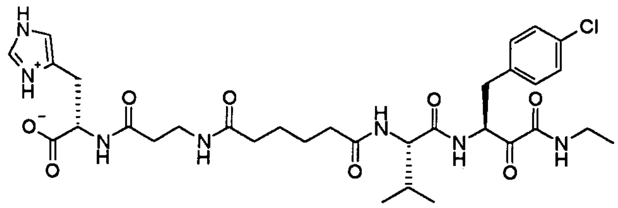

$R_f$ = 0.43 (MeOH/AcOH 9:1).

Example 33:

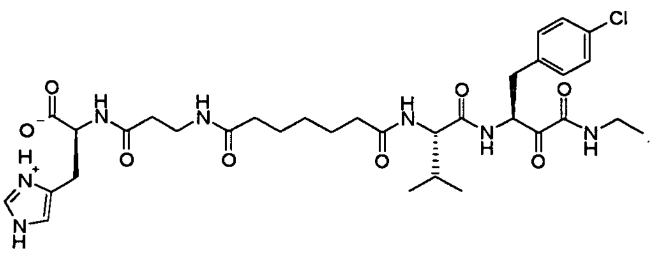

$R_f$ = 0.48 (MeOH/AcOH 9:1 ); Mp. 180-182 °C.

Example 34:

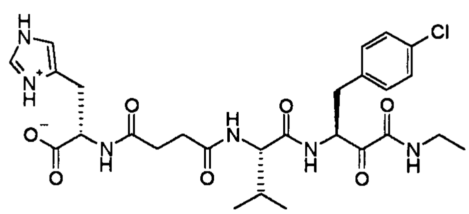

$R_f$ = 0.58 (MeOH/AcOH 9:1).

Example 35:

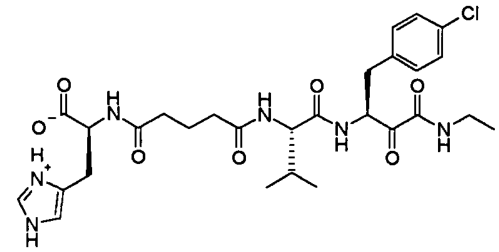

$R_f$ = 0.59 (MeOH/AcOH 9:1).

Example 36:

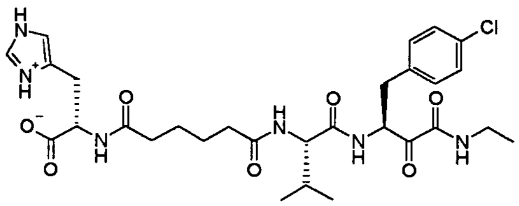

$R_f$ = 0.60 (MeOH/AcOH 9:1).

Example 37:

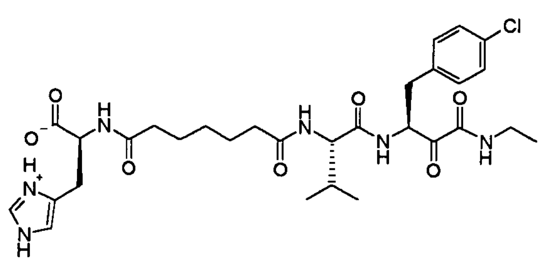

$R_f$ = 0.62 (MeOH/AcOH 9:1).

Example 38:

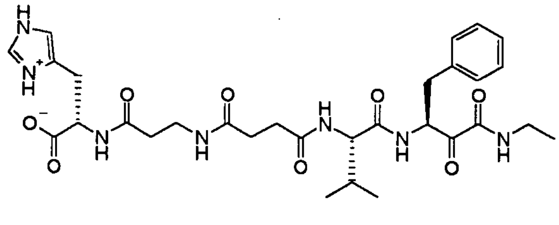

$R_f$ = 0.53 ($CHCl_3$/MeOH/AcOH 5:3:1), mp: 171 °C.

Example 39:

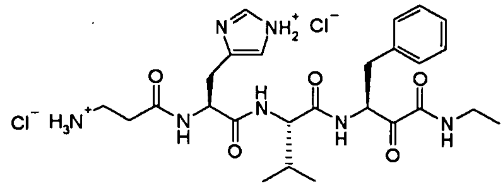

44 mg of the intermediate 39d) were dissolved in 5 ml of 4 M HCl in dioxane at 0 °C. The solution was stirred for 40 min. The volatile reagents were evaporated in vacuo while repeatedly adding toluene. The remaining, white solid was washed with 5 ml of dichloromethane and dried in vacuo.
$R_f$ = 0.33 (RP C-18 tlc-plate, $H_2O/CH_3CN$/1n HCl 1:1:0.1).

The required intermediates can be synthesized in the following way:

*Intermediate 39a):*

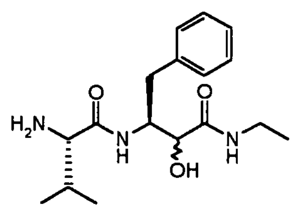

This intermediate was synthesized in analogy to intermediate 1 a) from Boc-phenylalaninal and Ethyl isocyanide.
$R_f$ = 0.30 ($CH_2Cl_2$/MeOH 60:5).

*Intermediate 39b):*

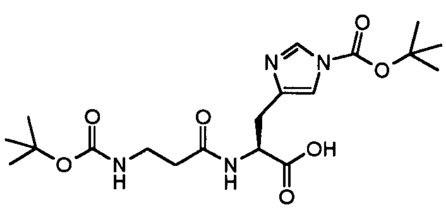

2 ml of 1 n NaOH was added to a suspension of 452 mg of L-Carnosine in 5 ml of dioxane/water 2:1. The resulting solution was cooled to 0 °C and treated with 960 mg of Di-tert-butyl dicarbonate. The reaction mixture was stirred for 40 min at rt. The dioxane was evaporated in vacuo and the residue wass diluted with 20 ml of iced water. 30 ml of cold ethyl acetate was added and the pH was adjusted to 2.2 by dropwise addition of 1n $KHSO_4$. The aqueous phase was extracted twice with 30 ml of ethyl acetate. The combined organic phases were washed with 20 ml of water and dried over $MgSO_4$. After filtration, the solvent was evaporated in vacuo to yield 664 mg of the di-boc protected product as a stable, colorless foam.

$R_f$ = 0.30 ($CH_2Cl_2$/MeOH 60:5).

*Intermediate 39c):*

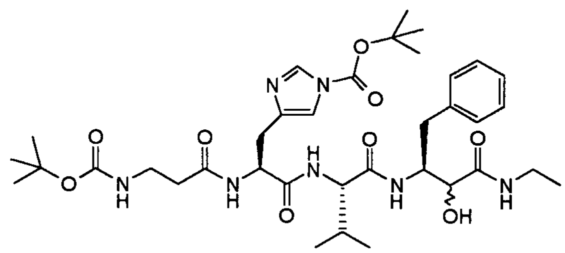

A solution of 222 mg of the intermediate 39b) in 5 ml of DMF was stirred at rt with 201 mg of HBTU and 128 µl of DIEA for 10 min. 160.5 mg of the intermediate XYa) was added and stirring was continued over night. The reaction mixture was slowly poured into 50 ml of 2n $K_2CO_3$ while stirring rapidly. After 40 min the precipitated product was collected by filtration, washed with water and dried. It was purified by flash chromatography on silica gel using dichloromethane/methanol 25:1 as eluent. $R_f$ = 0.29 ($CH_2Cl_2$/MeOH 60:5); Mp. 154°C (decomposition).

*Intermediate 39d):*

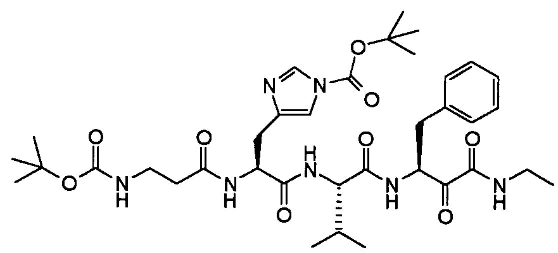

A solution of 108 mg of the peptidyl alcohol 39c) in a mixture of 6 ml dichloromethane and 0.3 ml of DMSO was cooled to 0 °C. 88.0 mg of Dess-Martin periodinane was added and the reaction mixture was stirred for 2 h while slowly warming up to roomtemperature. The solution was diluted with 50 ml of dichloromethane and extracted with 20 ml of 1n $Na_2S_2O_3$, 20 ml of 1n $NaHCO_3$ and 20 ml of water. The organic phase was separated, dried over $MgSO_4$, filtered and evaporated in vacuo.
$R_f$ = 0.35 ($CH_2Cl_2$/MeOH 60:5); Mp. 169 °C (decomposition).

The following examples can be prepared in a similar way:

Example 40:

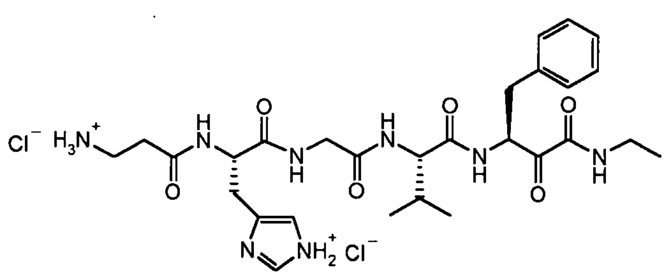

$R_f$ = 0.03 (RP C-18 tlc-plate, $H_2O/CH_3CN$ 1:2).

Example 41:

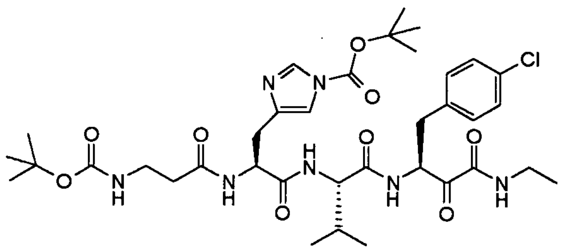

$R_f$ = 0.55 ($CH_2Cl_2$/MeOH 9:1); Mp. 196-200 °C.

Example 42:

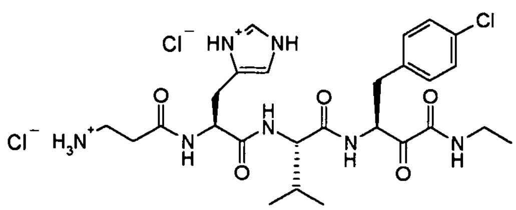

$R_f$ = 0.02 ($CH_2Cl_2$/MeOH/$Et_3N$ 9:1:0.2); Mp. 226-239 °C.

Example 43:

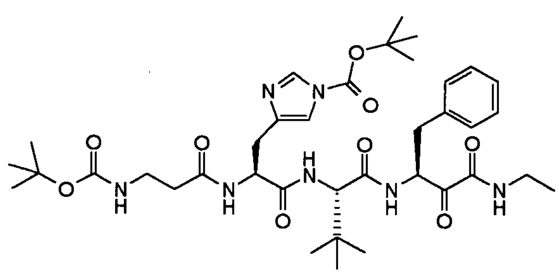

$R_f$ = 0.55 ($CH_2Cl_2$/MeOH 9:1).

Example 44:

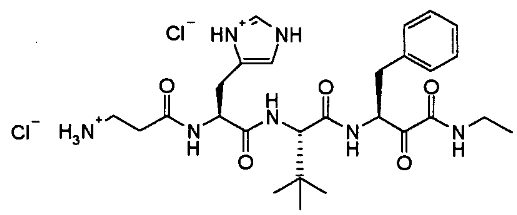

$R_f$ = 0.07 ($CH_2Cl_2$/MeOH 9:1); Mp. 187°C dec.

Example 45:

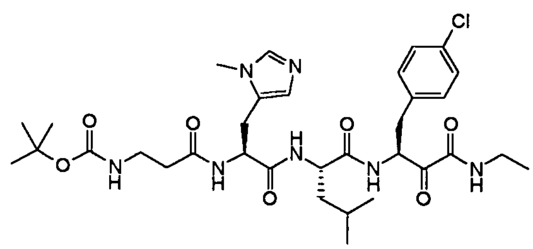

$R_f$ = 0.73 (MeOH/AcOH 9:1); Mp. 140-142 °C.

Example 46:

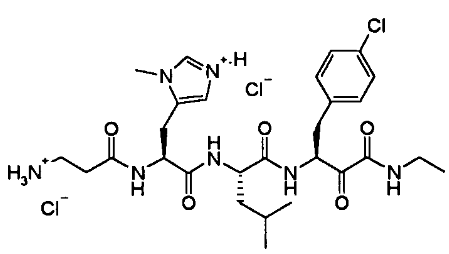

$R_f$ = 0.20 (MeOH/AcOH 9:1); Mp.180-185 °C.

Example 47:

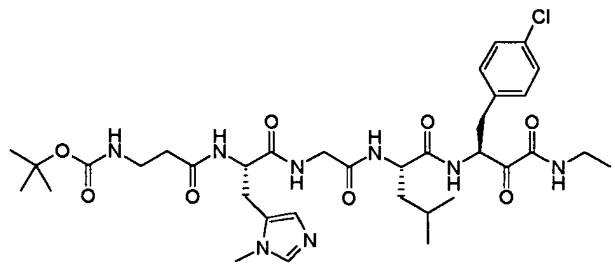

$R_f$ = 0.54 (MeOH/AcOH 9:1); Mp. 173-175 °C.

Example 48:

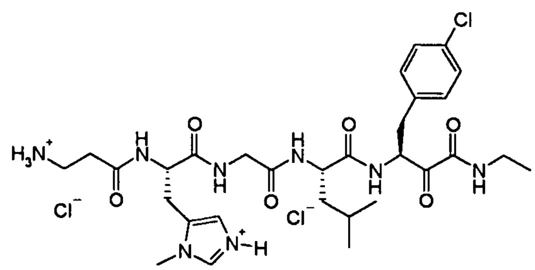

$R_f$ = 0.20 (MeOH/AcOH 9:1); Mp. 280 °C (decomposition).

Example 49:

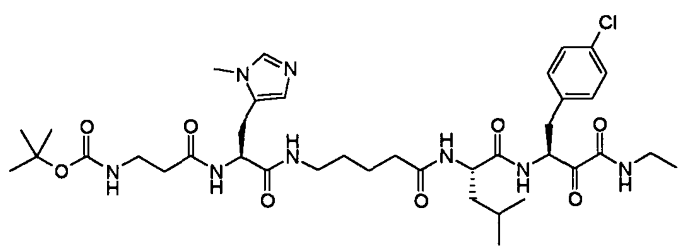

$R_f$ = 0.50 (MeOH/AcOH 9:1); Mp. 116-120 °C.

Example 50:

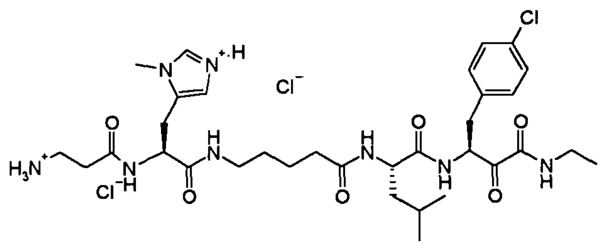

$R_f$= 0.19 (MeOH/AcOH 9:1); Mp.160-175 °C.

Example 51:

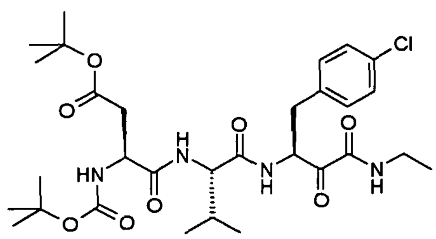

$R_f$= 0.52 ($CH_2Cl_2$/MeOH 9:1); Mp. 191-192 °C.

Example 52:

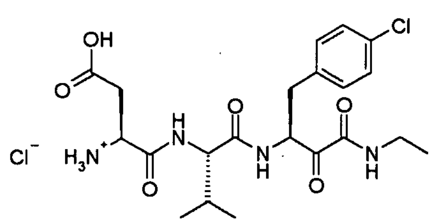

$R_f$ = 0.05 ($CH_2Cl_2$/MeOH/AcOH 6:1:0.1 ); Mp. 189-202 °C.

Example 53:

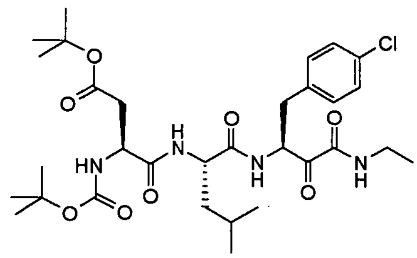

$R_f$ = 0.53 ($CH_2Cl_2$/MeOH 9:1); Mp. 165-166 °C.

Example 54:

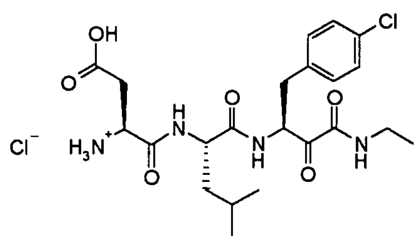

$R_f$ = 0.02 ($CH_2Cl_2$/MeOH 9:1); Mp. 185-187 °C.

Example 55:

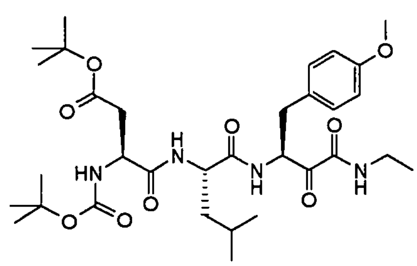

$R_f$ = 0.60 ($CH_2Cl_2$/MeOH 9:1); Mp. 171-172 °C.

Example 56:

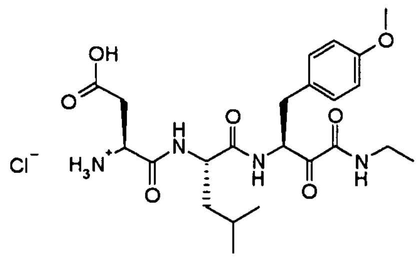

$R_f$ = 0.02 ($CH_2Cl_2$/MeOH 9:1); Mp. 158-159 °C.

Example 57:

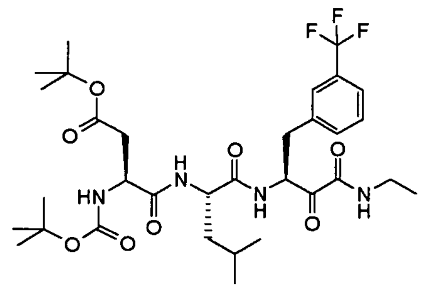

$R_f$ = 0.62 ($CH_2Cl_2$/MeOH 9:1); Mp. 192-194 °C.

Example 58:

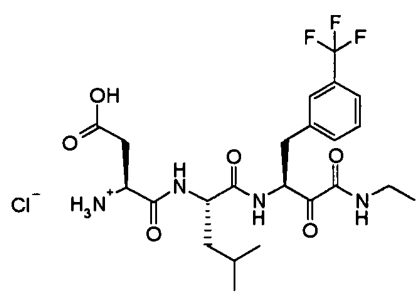

$R_f$ = 0.05 ($CH_2Cl_2$/MeOH 9:1); Mp. 165-168 °C.

Example 59:

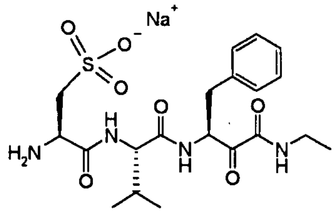

60 mg of the intermediate 59c) were dissolved in 5 ml of DMF. The solution was treated with 0.5 ml of piperidine. After 10 min the solvent and excess base were evaporated in high vacuum. The remaining white solid was suspended in 30 ml of 0.1 n HCl and extracted with 50 ml of ethyl acetate. The aqueous phase was evaporated and the residue was purified by C-18 reverse phase chromatography using an acetonitrile/water gradient for elution.
$R_f$ = 0.15 ($CH_2Cl_2$/MeOH/AcOH 5:1:0.1 ).

The required intermediates can be synthesized in the following way:

*Intermediate 59a):*

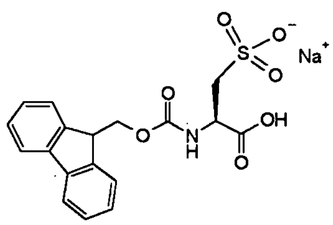

To a solution of 1.25 g cysteic acid in 5 ml of water was added 1 n NaOH until pH 8 was reached. A solution of 4.554 g of Fmoc-ONSu in 25 ml of dioxane was added in one portion while stirring rapidly. A fine colorless precipitate formed. Stirring was continued for 20 h at room temperature. The precipitate was filtered and discarded. The filtrate was evaporated in vacuo. After redissolution in 100 ml of water, it was extracted with 50 ml of ethyl acetate. The aqueous layer was separated, evaporated in vacuo and dried thoroughly by coevaporation with toluene. The crude product was digerated with 100 ml of abs. ethanol for 1 h at 60 °C. The suspension was cooled in an ice bath and the product was collected by filtration.
$R_f$ = 0.49 ($CHCl_3$/MeOH/33% AcOH 5:3:1).

*Intermediate 59b):*

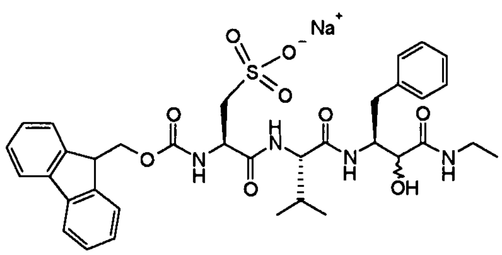

A suspension of the intermediate 59a) (269.5 mg) in 7 ml of DMF was reacted with 176.7 µl of diisopropylethylamine and 322.1 mg of PyBOP for 15 min. The aminoterminally deprotected dipeptide intermediate 39a) was

added and the reaction mixture was stirred over night at room temperature. The solvent was evaporated in high vacuum and the remaining solid was taken up in 50 ml of ethyl acetate. After extraction with 30 ml of 0.1 n HCl, the org. phase was dried over $MgSO_4$, filtered and evaporated. The remaining crude product was purified by silica gel chromatography with $CH_2Cl_2$/MeOH/AcOH 12:1:0.1.
$R_f$ = 0.13 ($CH_2Cl_2$/MeOH/AcOH 12:1:0.1), mp: 286 °C.

*Intermediate 59c):*

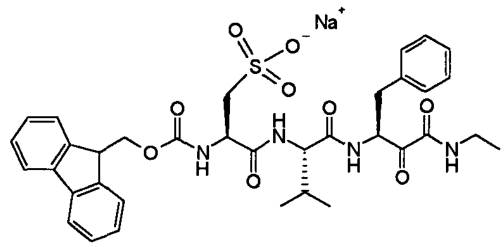

The peptidyl alcohol 59b) (112 mg) was suspended in a mixture of 5 ml of dichloromethane and 0.5 ml of DMSO. After cooling to 0 °C 99.3 mg of Dess Martin reagent was added. The reaction mixture was stirred for 3h while gradually warming to room temperature. The dichloromethane was evaporated and the residue was taken up in 40 ml of 0.5 n $Na_2S_2O_3$. The crude product precipitated as a colorless solid that was filtered, dried and further purified by silica gel chromatography with dichloromethane/methanol/acetic acid 10:1:0.1 to yield 68.3 mg of intermediate 59b).
The following examples can be prepared in a similar way:

Example 60:

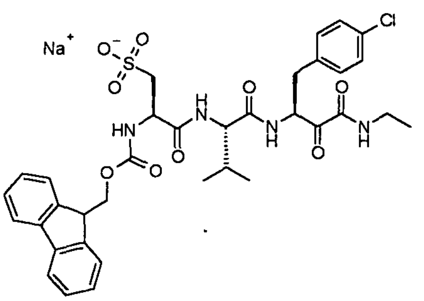

$R_f$ = 0.32 ($CH_2Cl_2$/MeOH/AcOH 9:1:0.1); Mp. 250 °C (decomposition).

Example 61:

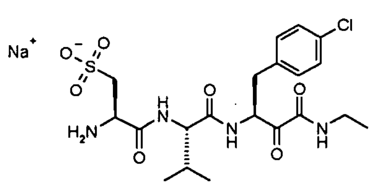

$R_f$ = 0.75 ($CHCl_3$/MeOH/$H_2O$/AcOH 50:30:7:3).

Biological Assays

The inhibiting effect of the a-keto carbonyl calpain inhibitors of formula (I) was determined using enzyme tests which are customary in the literature, with the concentration of the inhibitor at which 50% of the enzyme activity is inhibited (=$IC_{50}$) being determined as the measure of efficacy. The $K_i$ value was also determined in some cases. These criteria were used to measure the inhibitory effect of the compounds (I) on calpain I, calpain II and cathepsin B.

Enzymatic Calpain Inhibition Assay

The inhibitory properties of calpain inhibitors are tested in 100 µl of a buffer containing 100 mM imidazole pH 7.5, 5 mM L-Cystein-HCl, 5 mM $CaCl_2$, 250 µM of the calpain fluorogenic substrate Suc-Leu-Tyr-AMC (Sigma) (Sasaki et al. J. Biol. Chem. 1984, Vol. 259, 12489-12949) dissolved in 2.5 µl DMSO and 0.5 µg of human µ-calpain (Calbiochem). The inhibitors dissolved in 1 µl DMSO are added to the reaction buffer. The fluorescence of the cleavage product 7-amino-4-methylcoumarin (AMC) is followed in a SPECTRAmax GEMINI XS (Molecular Device) fluorimeter at $\lambda_{ex}$ = 360 nm and $\lambda_{em}$ = 440 nm at 30°C during 30 min at intervals of 30 sec in 96-well plates (Greiner). The initial reaction velocity at different inhibitor concentrations is plotted against the inhibitor concentration and the IC50 values determined graphically.

Calpain Inhibition Assay in C2C12 Myoblasts

This assay is aimed at monitoring the ability of the substance to inhibit cellular calpains. C2C12 myoblasts are grown in 96-well plates in growth medium (DMEM, 20% foetal calf serum) until they reach confluency. The growth medium is then replaced by fusion medium (DMEM, 5 % horse serum). 24 hours later the fusion medium is replaced by fusion medium containing the test substances dissolved in 1 µl DMSO. After 2 hours of incubation at 37°C the cells are loaded with the calpain fluorogenic substrate Suc-Leu-Tyr-AMC at 400 µM in 50 µl of a reaction buffer containing 135 mM NaCl; 5 mM KCl; 4 mM $CaCl_2$; 1 mM $MgCl_2$; 10 mM Glucose; 10 mM HEPES pH 7.25 for 20 min at room temperature. The calcium influx, necessary to activate the cellular calpains, is evoked by the addition of 50 µl reaction buffer containing 20 µM of the calcium ionophore Br-A-23187 (Molecular Probes). The fluorescence of the cleavage product AMC is measured as described above during 60 min at 37°C at intervals of 1 min. The IC50 values are determined as described above. Comparison of the IC50 determined in the enzymatic calpain inhibition assay to the IC50 determined in the C2C12 myoblasts calpain inhibiton assay, allows to evaluate the cellular uptake or the membrane permeability of the substance.

Spectrin Breakdown Assay in C2C12 Myoblasts

Although calpains cleave a wide variety of protein substrates, cytoskeletal proteins seem to be particularly susceptible to calpain cleavage. Specifically, the accumulation of calpain-specific breakdown products (BDP's) of the cytoskeletal protein alpha-spectrin has been used to monitor calpain activity in cells and tissues in many physiological and pathological conditions. Thus, calpain activation can be measured by assaying the proteolysis of the cytoskeletal protein alpha-spectrin, which produces a large (150 kDa), distinctive and stable breakdown product upon cleavage by calpains (Harris, A.S., D.E. Croall, and J.S. Morrow, The calmodulin-binding site in alpha-fodrin is near the calcium-dependent protease-I cleavage site. J Biol Chem, 1988. 263(30): p. 15754-61. Moon, R.T. and A.P. McMahon, Generation of diversity in nonerythroid spectrins. Multiple polypeptides are predicted by sequence analysis of cDNAs encompassing the coding region of human nonerythroid alpha-spectrin. J Biol Chem, 1990. 265(8): p. 4427-33. Vanderklish, P.W. and B.A. Bahr, The pathogenic activation of calpain: a marker and mediator of cellular toxicity and disease states. Int J Exp Pathol, 2000. 81(5): p. 323-39.). The spectrin breakdown assay is performed under the same conditions as in the C2C12 myoblast calpain inhibition assay described above, except that the fluorogenic substrate is omitted. After the 60 min incubation with the calcium inonophore, the cells are lysed in 50 µl of lysis buffer containing 80 mM Tris-HCl pH 6.8; 5 mM EGTA; 2 % SDS. The lysates are then probed on western blots using the monoclonal antibody mAb1622 (Chemicon). The activation of calpains is determined by measuring the ratio of the 150 kDa calpain-specific BDP to the intact 240 kDa alpha-spectrin band densitometrically.

Cathepsin B Test

Inhibition of cathepsin B was determined by a method which was similar to a method of S. Hasnain et al., J. Biol.Chem. 1993, 268, 235-40.

2µL of an inhibitor solution, prepared from inhibitor and DMSO (final concentrations: 100 µM to 0.01 µM) are added to 88 µL of cathepsin B (human liver cathepsin B (Calbiochem) diluted to 5 units in 500 µM buffer). This mixture

is preincubated at room temperature (25° C) for 60 min and the reaction is then starting by adding 10 µL of 10 mM Z-Arg-Arg-pNA (in buffer containing 10% DMSO). The reaction is followed at 405 nm for 30 min in a microtiter plate reader. The $IC_{50}$'s are then determined from the maximum slopes.

BSO Assay

Primary fibroblasts were derived from donors with molecular diagnosis for Friedreich Ataxia (FRDA) and control donors with no mitochondrial disease. Cell lines were obtained from Coriell Cell Repositories (Camden, NJ; catalog numbers GM04078, GM08402 and GM08399 respectively). All cell types were diagnosed on the molecular level for intronic GAA triplet repeat length of at least 400-450 repeats using a PCR-based method. Experiments were carried out as described (Jauslin et al. 2002, "A cellular model for Friedreich Ataxia reveals small-molecule glutathione peroxidase mimetcs as novel treatment strategy" Human Mol. Genetics, Vol.11, No. 4, 3055-3063): Cells were seeded in microtiter plates at a density of 4'000 cells per 100 □l in growth medium consisting of 25% (v/v) M199 EBS and 64% (v/v) MEM EBS without phenol red (Bioconcept, Allschwil, Switzerland) supplemented with 10% (v/v) fetal calf serum (PAA Laboratories, Linz, Austria), 100 U/ml penicillin, 100 □g/ml streptomycin (PAA Laboratories, Linz, Austria), 10 □g/ml insulin (Sigma, Buchs, Switzerland), 10 ng/ml EGF (Sigma, Buchs, Switzerland), 10 ng/ml bFGF (PreproTech, Rocky Hill, NJ) and 2 mM glutamine (Sigma, Buchs, Switzerland). The cells were incubated in the presence of various test compounds for 24 h before addition of L-buthionine-(S,R)-sulfoximine (BSO) to a final concentration of 1 mM. Cell viability was measured after the first signs of toxicity appeared in the BSO-treated controls (typically after 16 to 48 h). The cells were stained for 60 min at room temperature in PBS with 1.2 □M calceinAM and 4 □M ethidium homodimer (Live/Dead assay, Molecular Probes, Eugene, OR). Fluorescence intensity was measured with a Gemini Spectramax XS spectrofluorimeter (Molecular Devices, Sunnyvale, CA) using excitation and emission wavelengths of 485 nm and 525 nm respectively.

Examples of a Pharmaceutical Composition

As a specific embodiment of an oral composition of the present invention, 80 mg of the compound of Example 1 is formulated with sufficient finely divided lactose to provide a total amount of 580 to 590 mg to fill a size 0 hard gelatin capsule.

As another specific embodiment of an oral composition of a compound of the present invention, 100 mg of the compound of Example 7 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the scope of the invention. For example, effective dosages other than the preferred doses as set forth hereinabove may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

**1.** A compound of structural formula (I):

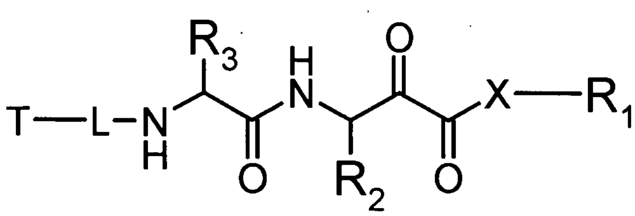

**(I)**

or a pharmaceutically acceptable salt or solvate thereof, wherein

$R^1$ represents

- hydrogen,
- straight chain alkyl,
- branched chain alkyl,
- cycloalkyl,
- -alkylene-cycloalkyl,
- aryl,
- -alkylene-aryl,
- $-SO_2$-alkyl,
- $-SO_2$-aryl,
- -alkylene-$SO_2$-aryl,
- -alkylene-$SO_2$-alkyl,
- heterocyclyl or
- -alkylene-heterocyclyl;

X represents O or NH;

$R^2$ represents

- hydrogen,
- straight chain alkyl,
- branched chain alkyl,
- cycloalkyl,
- -alkylene-cycloalkyl,
- aryl or
- -alkylene-aryl;

$R^3$ represents

- hydrogen,
- straight chain alkyl,
- branched chain alkyl,
- cycloalkyl or
- -alkylene-cycloalkyl;

L represents

a bond or at least one group selected from

- -CO- ,
- $-CO-(CH_2)_y-CO-$, wherein y is an integer of 1 to 6,
- $-NH-(CH_2)_z-CO-$ , wherein z is an integer of 1 to 6,
- -CO-cycloalkylene-CO- ,
- -NH-cycloalkylene-CO- ,
- -CO-arylene-CO- and
- -NH-arylene-CO-;

T is selected from the group consisting of

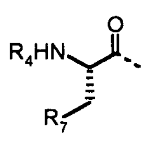

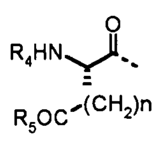

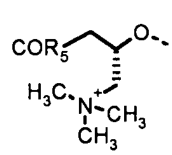

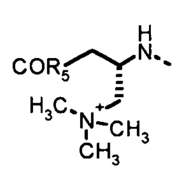

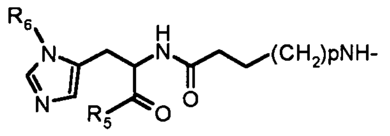
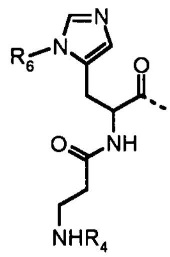
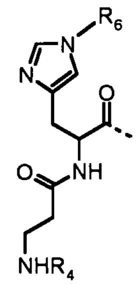
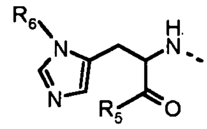
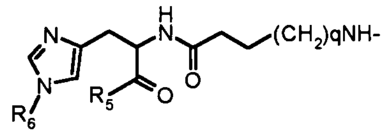
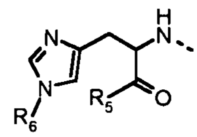

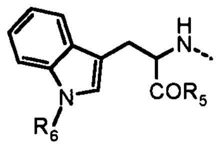
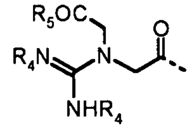
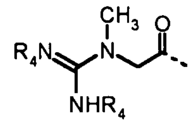
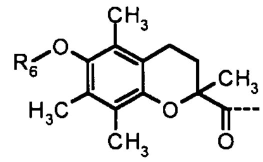

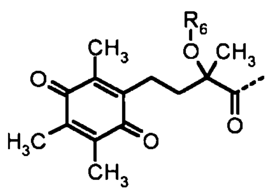
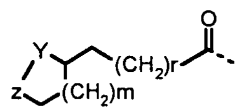
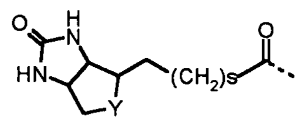
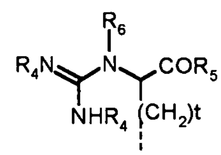

wherein each of m, n, p, q, r, s, and t represents an integer of 0 to 6;

$R^4$ represents
- hydrogen,
- $NH_2$-CO-,
- $NH_2$-CS-,
- $NH_2$-$SO_2$-,
- A-NH-CO-,
- $A_2$-N-CO-,
- A-NH-CS-,
- $A_2$-N-CS-,
- A-NH-$SO_2$-,
- $A_2$-N-$SO_2$-,
- A-CO-,
- A-CS-,
- A-$SO_2$-,
- A-O-CO- or
- A-O-CS-;

A is selected from the group consisting of
- straight chain or branched chain alkyl,
- straight chain or branched chain alkyl substituted with at least one halogen atom,
- straight chain or branched chain alkyl substituted with B,
- straight chain or branched chain alkyl substituted with at least one halogen atom and with B,
- cycloalkyl,
- cycloalkyl with at least one halogen atom,
- cycloalkyl substituted with B,
- cycloalkyl substituted with at least one halogen atom and with B,

straight chain or branched chain alkyl with an attached cycloalkyl group,
straight chain or branched chain alkyl with two attached cycloalkyl groups,
straight chain or branched chain alkyl with an attached cycloalkyl group substituted with B,
straight chain or branched chain alkyl with two attached cycloalkyl groups substituted with B,
1-adamantyl,
9-fluorenyl,
phenyl,
phenyl substituted with D,
phenyl disubstituted with D,
phenyl trisubstituted with D,
naphthyl,
naphthyl substituted with D,
naphthyl disubstituted with D,
naphthyl trisubstituted with D,
straight chain or branched chain alkyl with an attached phenyl group,
straight chain or branched chain alkyl with two attached phenyl groups,
straight chain or branched chain alkyl with an attached phenyl group substituted with D,
straight chain or branched chain alkyl with two attached phenyl groups substituted with D,
straight chain or branched chain alkyl with an attached phenoxy group,
straight chain or branched chain alkyl with an attached phenoxy group substituted with D on the phenoxy group, and
straight chain or branched chain alkyl with an attached 9-fluorenyl group;

B is selected from the group consisting of
halogen,
COOH,
OH,
CN,
$NO_2$,
$NH_2$,
alkoxy,
alkylamine,
dialkylamine,
alkyl-O-CO-,
alkyl-O-CO-NH- and
alkyl-S-;

D is selected from the group consisting of
halogen,
alkyl,
perfluoroalkyl,
alkoxy,
$NO_2$,
CN,
OH,
COOH,
$NH_2$,
alkylamino,
dialkylamino,
acyl,
alkyl-O-CO- and
alkyl-S-;

$R^5$ represents
XH,
$X^-$,
-X-alkyl,
-X-branched chain alkyl,

-X-cycloalkyl,
-X-alkylene-cycloalkyl,
-X-aryl,
-X-alkylene-aryl,
$-X-SO_2$-alkyl,
$-X-SO_2$-aryl,
-X-alkylene-$SO_2$-aryl,
-X-alkylene-$SO_2$-alkyl or
-X-alkylene-heterocyclyl,
wherein X is as defined above;

$R^6$ represents
hydrogen,
straight chain alkyl,
branched chain alkyl,
cycloalkyl,
-alkylene-cycloalkyl,
-alkylene-aryl, or
A-O-CO-,
A-NH-CO-
A-CO-
A-$SO_2$- or
A-NH-$SO_2$-
wherein A is as defined above;

$R^7$ represents
$-SO_3H$ or
$-SO_2NHR_6$,
wherein $R^6$ has the meaning as defined above; and

Y and Z independently represents
S,
SO or
$CH_2$.

**2.** The compound of claim 1, wherein $R^1$ is selected from the group consisting of straight chain or branched chain alkyl, cycloalkyl, -alkylene-cycloalkyl, -alkylene-aryl, -alkylene-heterocyclyl and -alkylene-$SO_2$-aryl.

**3.** The compound of claim 1 or 2, wherein $R^2$ is a substituted or unsubstituted benzyl group.

**4.** The compound of any of claims 1 to 3, wherein $R^3$ is a branched chain alkyl group, a cycloalkyl group or an -alkylene-cycloalkyl group.

**5.** The compound of any of claims 1 to 4, wherein L is a bond or a group selected from $-CO-(CH_2)_y-CO-$, wherein y is an integer of 1 to 6, -CO-cycloalkylene-CO,$-NH-(CH_2)_z-CO$, wherein z is an integer of 1 to 6, or -NH-cycloalkylene-CO- or a combination of two of these groups.

**6.** The compound of any of claims 1 to 5, wherein T is selected from the group consisting of

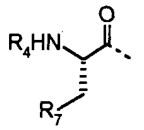 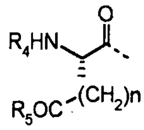 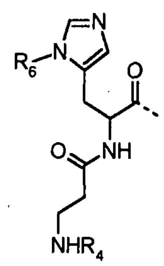 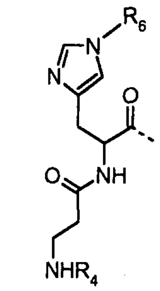 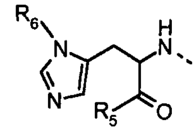

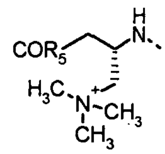 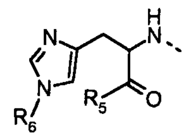

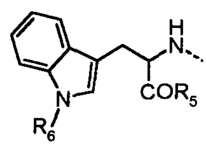 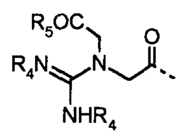 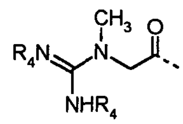 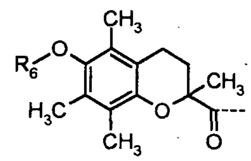

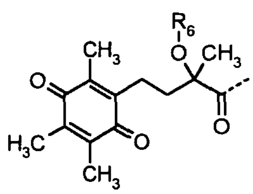 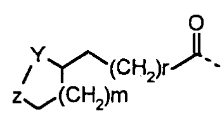 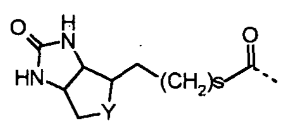 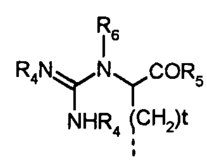

**7.** The compound of any of claims 1 to 5, wherein T is selected from the group consisting of

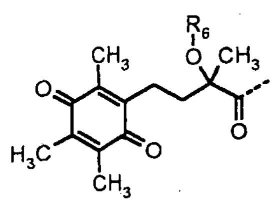 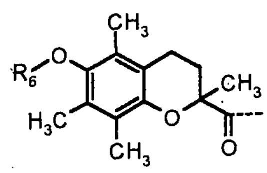

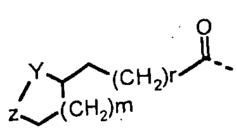 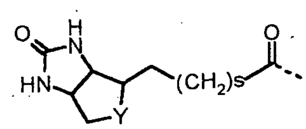

**8.** The compound of any of claims 1 to 7, wherein in each of groups T having a group $R^4$, $R^4$ is selected from the group consisting of A-O-CO-, A-NH-CO-, A-CO, and A-$SO_2$- wherein A is a straight chain alkyl group, which has 1 to 10 carbon atoms, a branched chain alkyl group, which has 3 to 10 carbon atoms, a cycloalkyl group, having 3 to 8 carbon atoms, an -alkylene-cycloalkyl group wherein the alkylene moiety is a straight chain alkylene group having 1 to 6 carbon atoms, and the cycloalkyl group has 3 to 8 carbon atoms, an aryl group, an -alkylene-aryl group, wherein the alkylene moiety is a straight chain alkylene group of 1 to 6 carbon atoms, and the aryl group is selected from substituted or unsubstituted phenyl, naphthyl, thienyl and pyridyl, or an -alkylene-heterocyclyl group wherein the alkylene moiety is a straight chain alkylene group of 1 to 6 carbon atoms.

9. The compound of any of claims 1 to 8, wherein $R^5$ is $-X-(CH_2)_xCH_3$, wherein x is an integer of 1 to 6; -X-branched chain alkyl having 3 to 6 carbon atoms, -X-cycloalkyl having 3 to 8 carbon atoms, -X-alkylene-cycloalkyl, wherein the alkylene moiety is a straight chain alkylene group having 1 to 6 carbon atoms, and the cycloalkyl group has 3 to 8 carbon atoms; -X-aryl selected from substituted or unsubstituted phenyl, naphthyl, thienyl and pyridyl, -X-alkylene-aryl, wherein the alkylene moiety is a straight chain alkylene group having 1 to 6 carbon atoms, and the aryl group is selected from substituted or unsubstituted phenyl, naphthyl, thienyl and pyridyl, -X-alkylene-heterocyclyl wherein the alkylene moiety is a straight chain alkylene group of 1 to 6 carbon atoms.

10. The compound of any of claims 1 to 9, wherein $R^6$ is hydrogen, a straight chain alkyl group having 1 to 6 carbon atoms, a branched chain alkyl group having 3 to 8 carbon atoms, a cycloalkyl group having 3 to 8 carbon atoms, an -alkylene-cycloalkyl group, wherein the alkylene moiety is a straight chain alkylene group having 1 to 6 carbon atoms, and the cycloalkyl group has 3 to 8 carbon atoms, an aryl group, an -alkylene-aryl group, wherein the alkylene moiety is a straight chain alkylene group of 1 to 6 carbon atoms, and the aryl group is selected from substituted or unsubstituted phenyl, naphthyl, thienyl and pyridyl, an -alkylene-heterocyclyl group, or A-O-CO-, A-NH-CO-, A-CO-, or $A-NH-SO_2-$, wherein A is a straight chain alkyl group, which has 1 to 10 carbon atoms, a branched chain alkyl group, which has 3 to 10 carbon atoms, a cycloalkyl group, having 3 to 8 carbon atoms, an -alkylene-cycloalkyl group wherein the alkylene moiety is a straight chain alkylene group preferably having 1 to 6 carbon atoms, and the cycloalkyl group has 3 to 8 carbon atoms, an aryl group, an -alkylene-aryl group, wherein the alkylene moiety is a straight chain alkylene group of 1 to 6 carbon atoms, and the aryl group is selected from substituted or unsubstituted phenyl, naphthyl, thienyl and pyridyl, or an -alkylene-heterocyclyl group wherein the alkylene moiety is a straight chain alkylene group of 1 to 6 carbon atoms.

11. The compound of any of claims 1 to 8, wherein $R^7$ is $SO_3NHR_6$.

12. The compound of any of claims 1 to 7, wherein Y and Z are both S or Y is S and Z is SO or Y is SO or Z is S.

13. The compound of any of claims 1 to 12 for use as a medicament.

14. Use of the compound of any of claims 1 to 13 for the preparation of a medicament for the treatment or prevention of disorders, diseases or conditions responsive to the inhibition of calpain I and other thiol proteases.

15. Use according to claim 14 for the preparation of a medicament for the treatment or prevention of disorders, diseases or conditions responsive to the inhibition of cathepsin B, cathepsin H, cathepsin L, or papain.

16. Use according to claim 14 for the preparation of a medicament for the treatment or prevention of disorders, diseases or conditions responsive to the inhibition of Multicatalytic Protease (MCP).

17. Use according to claim 14 for the preparation of a medicament for the treatment or prevention of Duchenne Muscular Dystrophy.

18. Use according to claim 14 for the preparation of a medicament for the treatment or prevention of Becker Muscular Dystrophy.

19. Use according to claim 14 for the preparation of a medicament for the treatment or prevention of neuromuscular diseases.

20. Use according to claim 19 for the preparation of a medicament for the treatment or prevention of muscular dystrophies, including dystrophinopathies and sarcoglycanopathies, limb girdle muscular dystrophies, congenital muscular dystrophies, congenital myopathies, distal and other myopathies, myotonic syndromes, ion channel diseases, malignant hyperthermia, metabolic myopathies, hereditary cardiomyopathies, congenital myasthenic syndromes, spinal muscular atrophies, hereditary ataxias, hereditary motor and sensory neuropathies and hereditary paraplegias.

21. Use according to claim 14 for the preparation of a medicament for the treatment or prevention of disuse atrophy and general muscle wasting.

22. Use according to claim 14 for the preparation of a medicament for the treatment or prevention of ischemias of the heart, of the kidney or of the central nervous system, inflammations, muscular dystrophies, cataracts of the eyes,

injuries to the central nervous system and Alzheimer's disease.

**23.** Use of the compounds of any of claims 1 to 13 for the preparation of a medicament for the treatment or prevention of mitochondrial disorders and neurodegenerative diseases, where elevated levels of oxidative stress are involved.

**24.** Use according to claim 23 for the preparation of a medicament for the treatment of mitochondrial disorders including, Kearns-Sayre syndrome, mitochondrial encephalomyopathy-lactic-acidosis-stroke like episodes (MELAS), myoclonic epilepsy and ragged-red-fibers (MERRF), Leber hereditary optic neuropathy (LHON), Leigh's syndrome, neuropathy-ataxia-retinitis pigmentosa (NARP) and progressive external opthalmoplegia (PEO).

**25.** Use according to claim 23 for the preparation of a medicament for the treatment of neurodegenerative diseases with free radical involvement including degenerative ataxias such as Friedreich' Ataxia, Parkinson's disease, Huntington's disease, amyotrophic lateral sclerosis (ALS) and Alzheimer's disease.

**26.** Use of the compounds of claim 7 for the preparation of a medicament for inhibiting cell damage by oxidative stress through free radicals.

**27.** Use of the compounds of claim 7 for the preparation of a medicament for the treatment of mitochondrial disorders and neurodegenerative diseases, where elevated levels of oxidative stress are involved.

**28.** A pharmaceutical composition which comprises a compound of any of claims 1 to 13 and a pharmaceutically acceptable carrier.

European Patent Office

# EUROPEAN SEARCH REPORT

Application Number
EP 03 00 4910

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 95 00535 A (ALKERMES INC ;HARBESON SCOTT L (US); STRAUB JULIE ANN (US)) 5 January 1995 (1995-01-05) * page 3, line 29 - page 4, line 3; claims 1-23; table I * --- | 1-28 | A61K38/05 A61K38/06 C07D339/04 C07K5/062 C07C237/22 C07D233/54 C07K5/023 C07K5/072 A61P25/28 A61P21/00 |
| Y | WO 92 12140 A (GEORGIA TECH RES INST) 23 July 1992 (1992-07-23) * page 6, line 8 - line 11; claims 1-15; tables III-IV * * page 34, line 3 - line 7 * --- | 1-28 | |
| Y | LI Z ET AL: "Novel Peptidyl alpha.-amide inhibitors of calpains and other cysteine proteases" JOURNAL OF MEDICINAL CHEMISTRY, AMERICAN CHEMICAL SOCIETY. WASHINGTON, US, vol. 39, 1996, pages 4089-4098, XP002180459 ISSN: 0022-2623 * the whole document * --- | 1-28 | TECHNICAL FIELDS SEARCHED (Int.Cl.7) |
| Y | US 5 514 694 A (CHU DER-LUN ET AL) 7 May 1996 (1996-05-07) * column 29, line 59 - line 61; claims 1-14 * --- | 1-28 | C07D C07K C07C |
| X | WO 99 64442 A (ANGELETTI P IST RICHERCHE BIO ;MARCHETTI ANTONELLA (IT); ONTORIA J) 16 December 1999 (1999-12-16) * page 16, line 4 - page 26, line 5; claims 1,2,13-17,26,27; table 2 * ----- | 1-6, 8-13,28 | |

~~The present search report has been drawn up for all claims~~

| Place of search | Date of completion of the search | Examiner |
|---|---|---|
| MUNICH | 31 July 2003 | Seymour, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 03 00 4910

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

subjects 1,3,4,8

☐ None of the further search fees have been paid within the fixed time limit. The present European search report has been drawn up for those parts of the European patent application which relate to the invention first mentioned in the claims, namely claims:

European Patent Office

**LACK OF UNITY OF INVENTION SHEET B**

Application Number
EP 03 00 4910

The Search Division considers that the present European patent application does not comply with the requirements of unity of invention and relates to several inventions or groups of inventions, namely:

1. Claims: 1-6 (part), 8-28 (part)

   Compounds of claim 1 where T is an amino acid R4NH-CH(CH2R7)-CO- or R4NH-CH((CH2)n-CO-R5)-CO- (see claim 1, definitions of T, row 1, residues 1 and 2) and the corresponding compositions and uses

2. Claims: 1-6 (part), 8-28 (part)

   Compounds of claim 1 where T bears a terminal trimethylammonium group (see claim 1, definitions of T, row 1, residues 3 and 4) and the corresponding compositions and uses

3. Claims: 1-6 (part), 8-28 (part)

   Compounds of claim 1 where T comprises a histidine moiety (see claim 1, definitions of T, rows 2 and 3) and the corresponding compositions and uses

4. Claims: 1-6 (part), 8-28 (part)

   Compounds of claim 1 where T comprises a tryptophan moiety (see claim 1, definitions of T, row 4, residue 1) and the corresponding compositions and uses

5. Claims: 1-6 (part), 8-28 (part)

   Compounds of claim 1 where T is a substituted glycine moiety (see claim 1, definitions of T, row 4, residues 2 and 3) and the corresponding compositions and uses

6. Claims: 1-28 (part)

   Compounds of claim 1 where T comprises a chroman moiety (see claim 1, definitions of T, row 4, residue 4) and the corresponding compositions and uses

7. Claims: 1-28 (part)

   Compounds of claim 1 where T comprises a benzoquinone moiety (see claim 1, definitions of T, row 5, residue 1) and the corresponding compositions and uses

European Patent Office

**LACK OF UNITY OF INVENTION SHEET B**

Application Number
EP 03 00 4910

The Search Division considers that the present European patent application does not comply with the requirements of unity of invention and relates to several inventions or groups of inventions, namely:

8. Claims: 1-28 (part)

   Compounds of claim 1 where T is a moiety (saturatated cycle)-(CH2)1-7-CO- (see claim 1, definitions of T, row 5, residues 2 and 3) and the corresponding compositions and uses

9. Claims: 1-6 (part), 7-28 (part)

   Compounds of claim 1 where T an alpha-amino acid bound through the alpha-carbon atom (see claim 1, definitions of T, row 5, residue 4) and the corresponding compositions and uses

## ANNEX TO THE EUROPEAN SEARCH REPORT ON EUROPEAN PATENT APPLICATION NO. EP 03 00 4910

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2003

| Patent document cited in search report | Publication date | Patent family member(s) | Publication date |
|---|---|---|---|
| WO 9500535 A | 05-01-1995 | US 5541290 A | 30-07-1996 |
| | | AU 7245294 A | 17-01-1995 |
| | | WO 9500535 A1 | 05-01-1995 |
| WO 9212140 A | 23-07-1992 | AU 654834 B2 | 24-11-1994 |
| | | AU 9155391 A | 17-08-1992 |
| | | CA 2098702 A1 | 29-06-1992 |
| | | EP 0564561 A1 | 13-10-1993 |
| | | JP 6504547 T | 26-05-1994 |
| | | WO 9212140 A1 | 23-07-1992 |
| | | US 5257901 A | 02-11-1993 |
| US 5514694 A | 07-05-1996 | NONE | |
| WO 9964442 A | 16-12-1999 | AU 754773 B2 | 21-11-2002 |
| | | AU 4279899 A | 30-12-1999 |
| | | CA 2330247 A1 | 16-12-1999 |
| | | EP 1084137 A1 | 21-03-2001 |
| | | WO 9964442 A1 | 16-12-1999 |
| | | JP 2002517508 T | 18-06-2002 |

EPO FORM P0459

For more details about this annex : see Official Journal of the European Patent Office, No. 12/82